(19) Europäisches Patentamt
European Patent Office
Office européen des brevets

(11) Veröffentlichungsnummer: **0 308 755 B1**

(12) **EUROPÄISCHE PATENTSCHRIFT**

(45) Veröffentlichungstag der Patentschrift: **28.10.92**

(21) Anmeldenummer: **88114847.2**

(22) Anmeldetag: **10.09.88**

Die Akte enthält technische Angaben, die nach dem Eingang der Anmeldung eingereicht wurden und die nicht in dieser Patentschrift enthalten sind.

(51) Int. Cl.5: **C07C 69/712**, A01N 37/38, C07D 213/79, C07C 255/13, C07C 235/32, C07C 67/31, A01N 43/40, C07C 43/295, C07C 41/01, C07D 213/64

# (54) (7-(Hetero)Aryloxy-naphthalin-2-yl-oxy)-alkancarbonsäure-Derivate.

(30) Priorität: **22.09.87 DE 3731801**

(43) Veröffentlichungstag der Anmeldung:
**29.03.89 Patentblatt 89/13**

(45) Bekanntmachung des Hinweises auf die Patenterteilung:
**28.10.92 Patentblatt 92/44**

(84) Benannte Vertragsstaaten:
**BE CH DE FR GB IT LI NL**

(56) Entgegenhaltungen:
**DE-A- 3 434 447**

**CHEMICAL ABSTRACTS, Band 107, 1987, Seite 7, Nr. 236243k, Columbus, Ohio, US;**

(73) Patentinhaber: **BAYER AG**

**W-5090 Leverkusen 1 Bayerwerk(DE)**

(72) Erfinder: **Haug, Michael, Dr.**
**Fahner Weg 5**
**W-5060 Bergisch-Gladbach 2(DE)**
Erfinder: **Andree, Roland, Dr.**
**Schillerstrasse 17**
**W-4018 Langenfeld(DE)**
Erfinder: **Santel, Hans-Joachim, Dr.**
**Grünstrasse 9 a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Strang, Harry, Dr.**
**Unterdorfstrasse 6 a**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue (7-(Hetero)Aryloxynaphthalin-2-yl-oxy)-alkancarbonsäure-Derivate, Verfahren und neue Zwischenprodukte zu ihrer Herstellung sowie ihre Verwendung als Herbizide.

Es ist bereits bekannt, daß bestimmte Dioxy-benzol-Derivate, wie z. B. α-(4-(2,4-Dichlor-phenoxy)-phenoxy)-propionsäure-methylester (Diclofop-methyl) herbizid wirksam sind (vgl. DE-OS 22 23 894). Des weiteren werden in der DE-OS-3 434 447 und C.A. 107, 236 243k 7-(Aryloxy)-2-Naphthyloxy-Alkancarbonsäurederivate, die eine herbizide Wirkung zeigen, beschrieben. Die Wirkung dieser bekannten Verbindungen gegen Unkräuter sowie ihre Kulturpflanzen-Verträglichkeit sind jedoch nicht immer zufriedenstellend.

Es wurden nun neue (7-(Hetero)Aryloxy-naphthalin-2-yl-oxy)-alkancarbonsäure-Derivate der allgemeinem Formel (I)

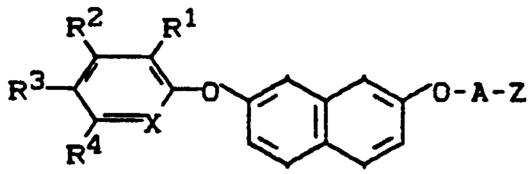

(I)

in welcher

$R^1$ für Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
X für Stickstoff oder die Gruppierung C-$R^5$ steht, worin
$R^5$ für Wasserstoff oder Halogen steht,

- mit der Maßgabe, daß wenigstens einer der Substituenten $R^2$ oder $R^4$ von Wasserstoff verschieden ist, wenn $R^1$ für Chlor oder Cyano und gleichzeitig $R^3$ für Trifluormethyl sowie X für CH steht und daß $R^3$ von Trifluormethyl verschieden ist, wenn X für Stickstoff steht -

A für gegebenenfalls verzweigtes Alkandiyl steht und
Z für Cyano oder die Gruppierung -CO-Y steht, worin
Y für Halogen, Hydroxy, Amino, Alkylamino, Alkylenamino, Alkinylamino, Arylamino, Aralkylamino, Alkoxycarbonylalkylamino, Cyanamino, Dialkylamino, Dialkenylamino, N-Alkylarylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Aralkoxyalkyl, Trialkylsilylalkyl, Arylthioalkyl, Aralkylthioalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall- oder Erdalkalimetall-äquivalent steht
oder für die Gruppierung

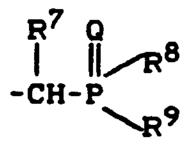

steht, worin
$R^7$ für Wasserstoff, Alkyl, Aryl, Furyl, Thienyl oder Pyridyl steht,
$R^8$ für Alkyl oder Alkoxy steht,
$R^9$ für Alkoxy steht und
Q für Sauerstoff oder Schwefel steht,
oder
$R^6$ für die Gruppierung $-(CH_2)_n-R^{10}$ steht, worin
$R^{10}$ für einen gegebenenfalls durch Halogen und/oder Alkyl substituierten heterocyclischen Rest aus

der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht,

gefunden.

Soweit die neuen (7-(Hetero)Aryloxy-naphthalin-2-yl-oxy)-alkancarbonsäure-Derivate asymmetrische Kohlenstoffatome enthalten, betrifft die Erfindung sowohl die einzelnen möglichen Isomeren als auch Gemische dieser Isomeren.

Bekannte Verbindungen wurden durch den in Formel (I) aufgestellten Disclaimer ausgenommen (vgl. EP-A-179 015 und JA 62/10041).

Weiter wurde gefunden, daß man die neuen (7-(Hetero)-Aryloxy-naphthalin-2-yl-oxy)-alkancarbonsäure-Derivate der Formel (I) erhält, wenn man

(a) 7-(Hetero)Aryloxy-2-naphthole der allgemeinen Formel (II)

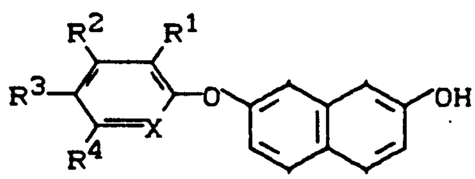

(II)

in welcher

$R^1$, $R^2$, $R^3$, $R^4$ und X die oben angegebenen Bedeutungen haben,

mit Carbonsäurederivaten der allgemeinen Formel (III)

$Z^1$ - A - Z (III)

in welcher

A und Z die oben angegebenen Bedeutungen haben und

$Z^1$ für eine nucleophile Abgangsgruppe steht,

gegebenenfalls in Gegenwart eines Säureakzeptors und gegebenenfalls in Gegenwart eines Verdünnungsmittels umsetzt, oder

(b) Halogen-(hetero)aryl-Verbindungen der allgemeinen Formel (IV)

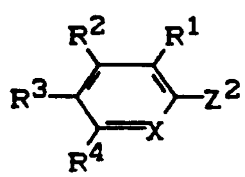

(IV)

in welcher

$R^1$, $R^2$, $R^3$, $R^4$ und X die oben angegebenen Bedeutungen haben und

$Z^2$ für Halogen steht,

mit (7-Hydroxy-naphthalin-2-yl-oxy)-alkancarbonsäure-Derivaten der allgemeinen Formel (V)

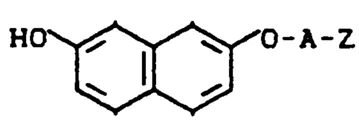

(V)

in welcher

A und Z die oben angegebenen Bedeutungen haben,

gegebenenfalls in Gegenwart eines Säureakzeptors und gegebenenfalls in Gegenwart eines Verdünnungsmittels umsetzt, oder

(c) für den Fall, daß in der Formel (I) Z für die Gruppierung -CO-Y steht, worin Y für Hydroxy steht und A, $R^1$, $R^2$, $R^3$, $R^4$ und X die oben angegebenen Bedeutungen haben, wenn man Verbindungen der Formel (I), in welcher Z für Cyano oder für die Gruppierung -CO-Y steht, worin Y für Methoxy oder Ethoxy steht und A, $R^1$, $R^2$, $R^3$, $R^4$ und Y die oben angegebenen Bedeutungen haben, mit einem Alkalihydroxid in Gegenwart eines organischen Lösungsmittels umsetzt und dann, gegebenenfalls nach Einengen, mit einer Mineralsäure ansäuert, oder

(d) für den Fall, daß in der Formel (I) Z für die Gruppierung -CO-Y steht, worin Y für Halogen steht und A, $R^1$, $R^2$, $R^3$, $R^4$ und X die oben angegebenen Bedeutungen haben, wenn man Verbindungen der allgemeinen Formel (I), in welcher Z für die Gruppierung -CO-Y steht, worin Y für Hydroxy steht und A, $R^1$, $R^2$, $R^3$, $R^4$ und X die oben angegebenen Bedeutungen haben, mit einem Halogenierungsmittel gegebenenfalls in Gegenwart eines Verdünnungsmittels umsetzt, oder

(e) für den Fall, daß Z in der Formel (I) für die Gruppierung -CO-Y steht, worin Y mit Ausnahme von Halogen die oben angegebene Bedeutung hat und A, $R^1$, $R^2$, $R^3$, $R^4$ und X die oben angegebenen Bedeutungen haben, wenn man Verbindungen der allgemeinen Formel (I), in welcher Z für Cyano oder die Gruppierung -CO-Y steht, worin Y für Halogen steht und A, $R^1$, $R^2$, $R^3$, $R^4$ und X die oben angegebenen Bedeutungen haben,
mit Verbindungen der allgemeinen Formel (VI)

H - Y (VI)

in welcher

Y mit Ausnahme von Halogen die oben angegebene Bedeutung hat,

gegebenenfalls in Gegenwart einer Säure bzw. eines Säureakzeptors und gegebenenfalls in Gegenwart eines Verdünnungsmittels umsetzt, oder

(f) für den Fall, daß Z in der Formel (I) für die Gruppierung -CO-Y steht, worin Y für die Gruppierung -O-$R^6$ steht, worin $R^6$ mit Ausnahme von Ammonium, Alkylammonium, Alkalimetall und Erdalkalimetall die oben angegebene Bedeutung hat und A, $R^1$, $R^2$, $R^3$, $R^4$ und X die oben angegebenen Bedeutungen haben, wenn man Verbindungen der allgemeinen Formel (I), in welcher Z für die Gruppierung -CO-Y steht, worin Y für Hydroxy steht und A, $R^1$, $R^2$, $R^3$, $R^4$ und Y die oben angegebenen Bedeutungen haben, mit Verbindungen der allgemeinen Formel (VII)

$Z^3$ - $R^{6-1}$ (VII)

in welcher

$R^{6-1}$ mit Ausnahme von Ammonium, Alkylammonium, Alkalimetall und Erdalkalimetall die oben für $R^6$ angegebene Bedeutung hat und

$Z^3$ für Halogen steht,

gegebenenfalls in Gegenwart eines Säureakzeptors und gegebenenfalls in Gegenwart eines Verdünnungsmittels umsetzt.

Schließlich wurde gefunden, daß die neuen (7-(Hetero)-Aryloxy-naphthalin-2-yl-oxy)-alkancarbonsäure-Derivate der allgemeinen Formel (I) starke herbizide Eigenschaften aufweisen.

Überraschenderweise zeigen die erfindungsgemäßen (7-(Hetero)Aryloxy-naphthalin-2-yl-oxy)-alkancarbonsäure-Derivate der Formel (I) bei guter Verträglichkeit in wichtigen Kulturpflanzen gegen Problemunkräuter wesentlich stärkere Wirkung als $\alpha$-(4-(2,4-Dichlorphenoxy)-phenoxy)-propionsäure-methylester, welches ein strukturell ähnlicher vorbekannter Wirkstoff gleicher Wirkungsrichtung ist.

Die Kohlenstoffketten in den einzelnen Resten, wie beispielsweise Alkyl, Alkoxy, Alkylthio, Alkoxyalkyl, Alkenyl, Alkinyl oder Alkylsulfinylalkyl sind jeweils geradkettig oder verzweigt. Die Substitution kann bei Verknüpfung der Rest mit "und/oder" jeweils einfach oder mehrfach, gleich oder verschieden erfolgen.

Die Erfindung betrifft vorzugsweise Verbindungen der Formel (I), in welcher

$R^1$ für Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder chlor steht,

X für Stickstoff oder die Gruppierung C-$R^5$ steht, worin

$R^5$ für Wasserstoff, Fluor, Chlor oder Brom steht,

- mit der Maßgabe, daß wenigstens einer der Substituenten $R^2$ oder $R^4$ von Wasserstoff verschieden ist, wenn $R^1$ für Chlor oder Cyano und gleichzeitig $R^3$ für Trifluormethyl sowie X für CH steht und daß $R^3$ von

Trifluormethyl verschieden ist, wenn X für Stickstoff steht -

A für gegebenenfalls verzweigtes $C_1$-$C_4$-Alkandiyl steht und

Z für Cyano oder die Gruppierung -CO-Y steht, worin

Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, N-($C_1$-$C_4$-alkyl)-phenylamino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxycarbonyl-$C_1$-$C_2$-alkylthio oder für die Gruppierung -O-$R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Tri-($C_1$-$C_2$-alkyl)-silyl-$C_1$-$C_2$-alkyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylaminocarbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- oder Calcium-äquivalent steht, oder für die Gruppierung

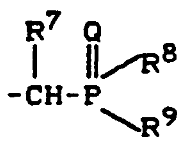

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ für die Gruppierung -$(CH_2)_n$-$R^{10}$ steht, worin,

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der Formel (I), in welcher

$R^1$ für Fluor, Chlor oder Trifluormethyl steht,

$R^2$ für Wasserstoff, Fluor oder Chlor steht,

$R^3$ für Chlor, Trifluormethyl oder Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor oder Chlor steht,

- mit der Maßgabe, daß wenigstens einer der Substituenten $R^2$ oder $R^4$ von Wasserstoff verschieden ist, wenn $R^1$ für Chlor und gleichzeitig $R^3$ für Trifluormethyl sowie X für CH steht und daß $R^3$ von Trifluormethyl verschieden ist, wenn X für Stickstoff steht -

A für Methylen (-$CH_2$-), Dimethylen (-$CH_2CH_2$-), Trimethylen (-$CH_2$-)$_3$

**Ethyliden** 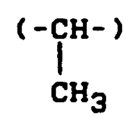 **oder**

**Propyliden** 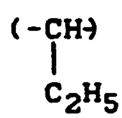

steht und

Z für Cyano oder die Gruppierung -CO-Y steht, worin

Y für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-($C_1$-$C_3$-alkyl)-amino, Diallylamino, N-Methylphenylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxycarbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium- oder Kalium-äquivalent steht, oder für die Gruppierung

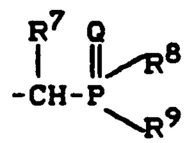

steht, worin

$R^7$ für Wasserstoff, Methyl, Phenyl, Furyl, Thienyl oder Pyridyl steht,

$R^8$ für Methoxy oder Ethoxy steht,

$R^9$ für $C_1$-$C_4$-Alkoxy steht und

Q für Sauerstoff oder Schwefel steht

oder

$R^6$ für die Gruppierung $-(CH_2)_n-R^{10}$ steht, worin

n für die Zahlen 0, 1 oder 2 steht und

$R^{10}$ für einen gegebenenfalls durch Chlor und/oder Methyl subtituierten heterocyclischen Rest aus der Reihe Furyl, Tetrahydrofuryl, Thienyl, Perhydropyranyl, Oxazolyl, Thiazolyl und Dioxolanyl steht.

Verwendet man für das erfindungsgemäße Verfahren (a) beispielsweise 7-(3,5-Dichlor-pyridin-2-yl-oxy)-2-naphthol und α-Brom-propionsäure-ethylester als Ausgangsstoffe, so kann der Reaktionsablauf durch das folgende Formelschema wiedergegeben werden:

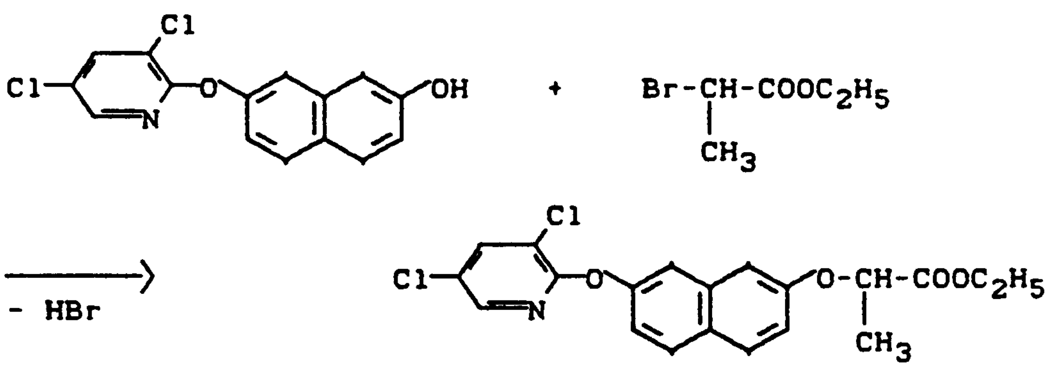


Verwendet man für das erfindungsgemäße Verfahren (b) beispielsweise 3,4,5-Trichlor-benzotrifluorid und (7-Hydroxy-naphthalin-2-yl-oxy)-essigsäure-butylester als Ausgangsstoffe, so kann der Reaktionsablauf durch das folgende Formelschema wiedergegeben werden:

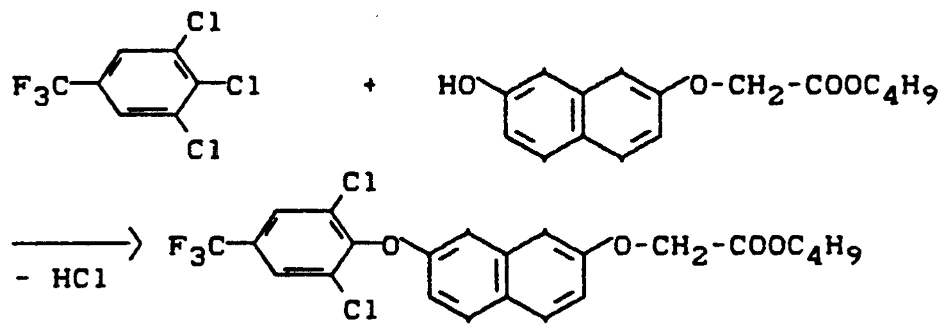


Verwendet man für das erfindungsgemäße Verfahren (c) beispielsweise β-(7-(3,5-Dichlor-pyridin-2-yl-oxy)-naphthalin-2-yl-oxy)-propionsäure-methylester und Natronlauge als Ausgangsstoffe, so kann der Reaktionsablauf durch das folgende Formelschema wiedergegeben werden:

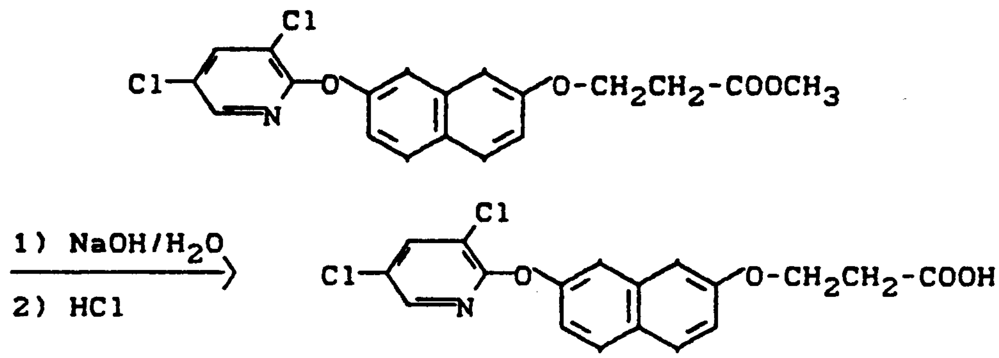


Verwendet man für das erfindungsgemäße Verfahren (d) beispielsweise α-(7-(2,6-Dichlor-3-fluor-4-trifluormethyl-phenoxy)-naphthalin-2-yl-oxy)-propionsäure und Thionylchlorid als Ausgangsstoffe, so kann der Reaktionsablauf durch das folgende Formelschema wiedergegeben werden:

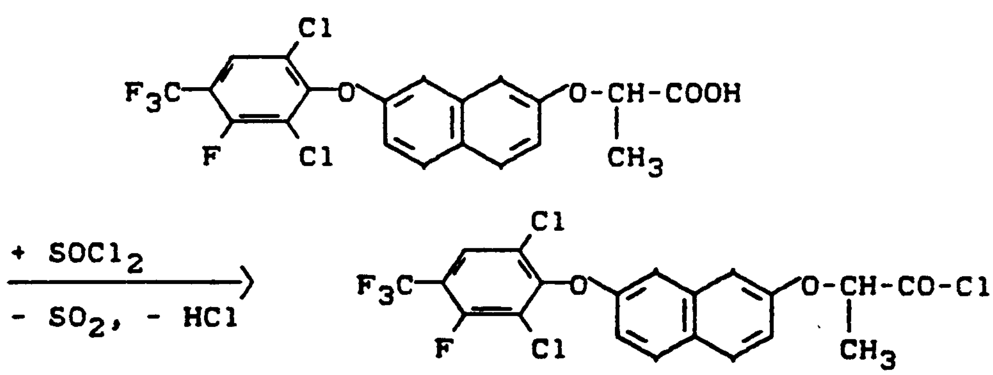


Verwendet man für das erfindungsgemäße Verfahren (e) beispielsweise α-(7-(2-Chlor-6-fluor-4-trifluormethylphenoxy)-naphthalin-2-yl-oxy)-propionsäure-chlorid und Mercaptoessigsäuremethylester als Ausgangsstoffe, so kann der Reaktionsablauf durch das folgende Formelschema wiedergegebenen werden:

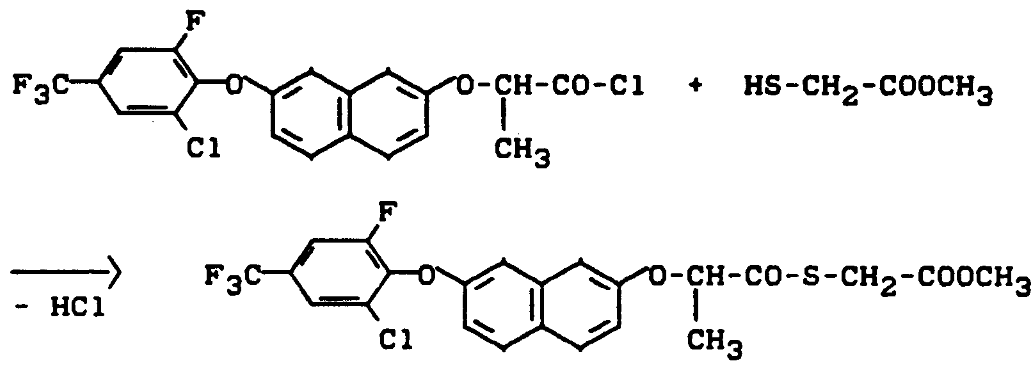


Verwendet man für das erfindungsgemäße Verfahren (f) beispielsweise (7-(2,3,6-Trichlor-4-trifluormethyll-phenoxy)-naphthalin-2-yl-oxy)-essigsäure und Trimethylsilylmethylchlorid als Ausgangsstoffe, so kann der Reaktionsablauf durch das folgende Formelschema wiedergegeben werden:

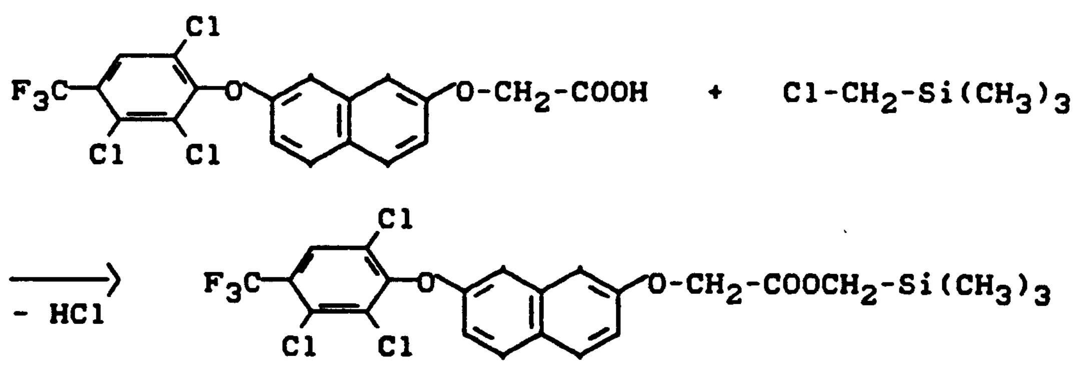


Die beim erfindungsgemäßen Verfahren (a) zur Herstellung von Verbindungen der Formel (I) als Ausgangsstoffe zu verwendenden 7-(Hetero)Aryloxy-2-naphthole sind durch die Formel (II) allgemein definiert.

In Formel (II) haben $R^1$, $R^2$, $R^3$, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Als Beispiele für die Ausgangsstoffe der Formel (II) seien genannt:
7-(2,6-Dichlor-4-trifluormethyl-phenoxy)-2-naphthol, 7-(2-Chlor-6-fluor-4-trifluormethyl-phenoxy)-2-naphthol, 7-(2,6-Dichlor-3-fluor-4-trifluormethyl-phenoxy)-2-naphtol, 7-(2,3,6-Trichlor-4-trifluormethyl-phenoxy)-2-naphthol und 7-(3,5-Dichlorpyridin-2-yl-oxy)-2-naphthol.

Die Ausgangsstoffe der Formel (II) sind teilweise aus der Literatur bekannt (vgl. EP-A 179 015 und JA 62/10041).

Noch nicht bekannt sind 7-(Hetero)Aryloxy-2-naphthole der Formel (IIa),

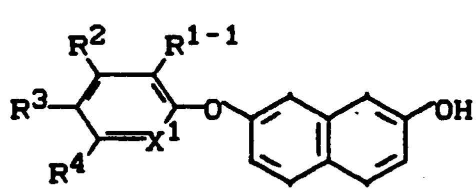
(IIa)

in welcher

| | |
|---|---|
| $R^{1-1}$ | für Halogen oder Trifluormethyl steht, |
| $R^2$ | für Wasserstoff oder Halogen steht, |
| $R^3$ | für Halogen, Trifluormethyl, Trifluormethoxy, Trifluormethylthio oder Trifluormethylsulfonyl steht, |
| $R^4$ | für Wasserstoff oder Halogen steht und |
| $X^1$ | für Stickstoff oder die Gruppierung $C\text{-}R^{5-1}$ steht, worin |

$R^{5-1}$ für Halogen steht.

Man erhält die Verbindungen der Formel (IIa), wenn man entsprechende Halogen(hetero)aryl-Verbindungen der Formel (IVa),

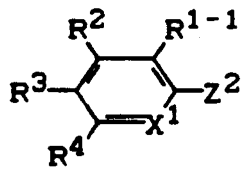

(IVa)

in welcher

$R^{1-1}$,$R^2$, $R^3$, $R^4$, $X^1$ und $Z^2$ die oben angegebenen Bedeutungen haben,

mit 2,7-Dihyroxynaphthalin in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet.

Bevorzugt werden die Verbindungen der Formel (IIa), in welcher

$R^{1-1}$ für Fluor, Chlor, Brom oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht und
$X^1$ für Stickstoff oder die Gruppierung $C=R^{5-1}$ steht, worin
$R^{5-1}$ für Fluor, Chlor oder Brom steht.

Besonders bevorzugt sind diejenigen Verbindung der Formel (IIa), in welcher

$R^{1-1}$ für Fluor oder Chlor steht,
$R^2$ für Wasserstoff, Fluor oder Chlor steht,
$R^3$ für Chlor oder Trifluormethyl steht,
$R^4$ für Wasserstoff, Fluor oder Chlor steht und
$X^1$ für Stickstoff oder die Gruppierung C-$R^{5-1}$ steht, worin
$R^{5-1}$ für Fluor oder Chlor steht.

Die Halogen-(hetero)aryl-Verbindungen sind durch die Formel (IVa) allgemein definiert und fallen unter die allgemeine Formel (IV), welche weiter unten beschrieben werden. In Formel (IVa) haben $R^{1-1}$, $R^2$, $R^3$, $R^4$ und $X^1$ vorzugsweise bzw. insbesondere diejenigen Bedeutungen, die bereits im Zusammenhang mit der Beschreibung der erfindungsgemäßen Verbindungen der Formel (IIa) vorzugsweise bzw. als insbesondere bevorzugt für $R^{1-1}$, $R^2$, $R^3$, $R^4$ und $X^1$ angegeben wurden und $Z^2$ steht vorzugsweise für Chlor oder Fluor.

Die beim erfindungsgemäßen Verfahren (a) weiter als Ausgangsstoffe zu verwendenden Carbonsäurederivate sind durch die Formel (III) allgemein definiert. In Formel (III) haben A und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt angegeben wurden und $Z^1$ steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methylphenylsulfonyloxy.

Als Beispiele für die Verbindungen der Formel (III) seien genannt:

Chloracetonitril, Bromacetonitril, β-Brom-propionitril, β-Chlor-propionitril, α-Chlor-, α-Brom- und α-Iod-propionsäure-methylester, -ethylester, -propylester, -isopropylester, -butylester, -isobutylester und -sec-butylester, β-Chlor-, β-Brom- und β-Iod-propionsäure-methylester, -ethylester, -propylester, -isopropylester, -butylester, -isobutylester und -sec-butylester, Chlor-, Brom- und Iod-essigsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Die Ausgangsstoffe der Formel (III) sind bekannt und/ oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird

vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Dexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden.

Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man je Mol 7-(Hetero)Aryloxy-2-naphthol der Formel (II) im allgemeinen zwischen 0,5 und 1,2 Mol, vorzugsweise zwischen 0,7 und 1,0 Mol Carbonsäurederivat der Formel (III) ein. Die Reaktionskomponenten werden im allgemeinen bei Raumtemperatur oder unter leichtem Kühlen zusammengegeben und dann - gegebenenfalls bei erhöhter Temperatur - bis zum Ende der Umsetzung gerührt.

Die Aufarbeitung kann auf übliche Weise durchgeführt werden. Beispielsweise wird das Reaktionsgemisch mit einer Säure, wie z. B. Salzsäure oder Schwefelsäure und Wasser, sowie einem mit Wasser praktisch nicht mischbaren organischen Lösungsmittel, wie z. B. Methylenchlorid oder Toluol, verrührt oder geschüttelt, die organische Phase abgetrennt, mit Wasser gewaschen, getrocknet und filtriert. Das nach Einengen des Filtrats im Rückstand verbleibende Produkt der Formel (I) kann auf übliche Weise, beispielsweise durch Säulenchromatographie, gereinigt werden.

Die beim erfindungsgemäßen Verfahren (b) als Ausgangsstoffe zu verwendenden Halogen-(hetero)aryl-Verbindungen sind durch die Formel (IV) allgemein definiert. In Formel (IV) haben $R^1$, $R^2$, $R^3$, $R^4$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden und $Z^2$ steht vorzugsweise für Chlor oder Fluor.

Als Beispiele für die Halogen-(hetero)aryl-Verbindungen der Formel (IV) seien genannt:
3,4,5-Trichlor-benzotrifluorid, 3,4-Dichlor-5-fluorbenzotrifluorid, 2,3,4,5-tetrachlor-benzotrifluorid, 3,5-Dichlor-2,4-difluor-benzotrifluorid, 3-Chlor-4,5-difluor-benzotrifluorid und 2,3,5-Trichlor-pyridin.

Die Verbindungen der Formel (IV) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x)).

Die beim erfindungsgemäßen Verfahren (b) weiter als Ausgangsstoffe zu verwendenden (7-Hydroxy-naphthalin-2-yl-oxy)-alkancarbonsäure-Derivate sind durch die Formel (V) allgemein definiert. In Formel (V) haben A und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt angegeben wurden.

Als Beispiele für die Ausgangsstoffe der Formel (V) seien genannt:
α-(7-Hydroxy-naphthalin-2-yl-oxy)-propionsäure-nitril, -methylester, -ethylester, -propylester, -isopropylester, -butylester, -isobutylester und -sec-butylester,
β-(7-Hydroxy-naphthalin-2-yl-oxy)-propionsäure-nitril, -methylester, -ethylester, -propylester, -isopropylester, -butylester, -isobutylester und -sec-butylester,
(7-Hydroxy-naphthalin-2-yl-oxy)-essigsäure-nitril, -methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. EP-A 179 015).

Verfahren (b) wird vorzugsweise unter Verwendung eines Verdünnungsmittels durchgeführt. Es kommen

vor allem diejenigen Verdünnungsmittel in Betracht, die bereits bei der Beschreibung des erfindungsgemäßen Verfahrens (a) genannt wurden. Aprotisch polare organische Lösungsmittel, wie z. B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Verfahren (b) wird vorzugsweise in Gegenwart eines Säureakzeptors duchgeführt. Es kommen vor allem diejenigen Säureakzeptoren in Betracht, die bereits bei der Beschreibung des erfindungsgemäßen Verfahren (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahren (b) setzt man je Mol Halogen-(hetero)aryl-Verbindung der Formel (IV) im allgemeinen zwischen 0,5 und 2 Mol vorzugsweise zwischen 0,7 und 1,5 Mol, (7-Hydroxy-naphthalin-2-yl-oxy)-alkancarbonsäure-Derivat der Formel (V) ein.

Umsetzung und Aufarbeitung können wie oben für Verfahren (a) beschrieben durchgeführt werden.

Die beim erfindungsgemäßen Verfahren (c) als Ausgangsstoffe zu verwendenden Verbindungen sind durch die Formel (I) mit der Maßgabe, daß Z für Cyano oder die Gruppierung -CO-Y steht, worin Y für Methoxy oder Ethoxy steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangstoffe zu Verfahren (c) seien genannt:

α-(7-(3,5-Dichlor-pyridin-2-yl-oxy)-, α-(7-(2,6-Dichlor-4-trifluormethyl-phenoxy), α-(7-(2-Chlor-6-fluor-4-trifluormethyl-phenoxy)-, α-(7-(2,3,6-Trichlor-4-trifluormethyl-phenoxy)- und α-(7-(2,6-Dichlor-3-fluor-4-trifluormethyl-phenoxy)-naphthalin-2-yl-oxy)-propionsäure-nitril, -methylester und ethylester;

β-(7-(3,5-Dichlor-pyridin-2-yl-oxy)-, β-(7-(2,6-Dichlor-4-trifluormethyl-phenoxy)-, β-(7-(2-Chlor-6-fluor-4-trifluormethyl-phenoxy)-, β-(7-(2,3,6-Trichlor-4-trifluormethyl-phenoxy)- und β-(7-(2,6-Dichlor-3-fluor-4-trifluormethyl-phenoxy)-naphthalin-2-yl-oxy)-propionsäure-nitril, -methylester und -ethylester;

(7(3,5-Dichlor-pyridin-2-yl-oxy)-, (7-(2-Chlor-6-fluor-4-trifluormethyl-phenoxy)-, (7-(2,3,6-Trichlor-4-trifluormethyl-phenoxy)- und (7-(2,6-Dichlor-3-fluor-4-trifluormethyl-phenoxy)-naphthalin-2-yl-oxy)-essigsäure-nitril, -methylester und -ethylester.

Die oben beschriebenen Ausgangsstoffe der Formel (I) für Verfahren (c) sind erfindungsgemäße, neue Verbindungen; sie können nach den erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegebenenfalls bei erhöhter Temperatur, bis zum Reaktionsende gerührt. Das - gegebenenfalls nach Einengen, Abkühlen und Ansäuern - kristallin anfallende Reaktionsprodukt kann durch Absaugen isoliert werden.

Die beim erfindungsgemäßen Verfahren (d) als Ausgangsstoffe zu verwendenden Verbindungen sind durch die Formel (I) mit der Maßgabe, daß Z für die Gruppierung -CO-Y steht, worin Y für Hydroxy steht, allgemein definiert, In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (d) seien genannt:

α-(7-3,5-Dichlor-pyridin-2-yl-oxy)-, α-(7-(2,6-Dichlor-4-trifluormethyl-phenoxy), α-(7-(2-Chlor-6-fluor-4-trifluormethyl-phenoxy)-, α-(7-(2,3,6-Trichlor-4-trifluormethyl-phenoxy)- und α-(7-(2,6-Dichlor-3-fluor-4-trifluormethyl-phenoxy)-naphthalin-2-yl-oxy)-propionsäure,
β-(7-(3,5-Dichlor-pyridin-2-yl-oxy)-, β-(7-(2,6-Dichlor-4-trifluormethyl-phenoxy)-, β-(7-(2-Chlor-6-fluor-4-trifluormethyl-phenoxy)-, β-(7-(2,3,6-Trichlor-4-trifluormethyl-phenoxy)- und β-(7-(2,6-Dichlor-3-fluor-4-trifluormethyl-phenoxy)-naphthalin-2-yl-oxy)-propionsäure,
(7(3,5-Dichlor-pyridin-2-yl-oxy)-, (7-(2,6-Dichlor-4-trifluormethyl-phenoxy)-, (7-(2-Chlor-6-fluor-4-trifluormethyl-phenoxy)-, (7-(2,3,6-Trichlor-4-trifluormethyl-phenoxy)- und (7-(2,6-Dichlor-3-fluor-4-trifluormethyl-phenoxy)-naphthalin-2-yl-oxy)-essigsäure,

Die oben beschriebenen Ausgangsstoffe der Formel (I) für Verfahren (d) sind erfindungsgemäße, neue Verbindungen; sie können nach den erfindungsgemäßen Verfahren (c) hergestellt werden.

Verfahren (d) wird unter Verwendung eines Halogenierungsmittels durchgeführt. Es können die üblichen Mittel für den Umsetzung von Carbonsäuren zur Carbonsäurehalogeniden eingesetzt werden. Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (d) wird gegebenenfalls in Gegenwart eines Katalysators durchgeführt. Es können die für die Herstellung von Säurechloriden aus Säuren üblichen Katalysatoren, wie z. B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß Z für Cyano oder die Gruppierung -CO-Y steht, worin Y für Halogen steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Y steht vorzugsweise für Fluor, Chlor oder Brom, insbesondere für Chlor.

Als Beispiele für die Ausgangsstoffe zu Verfahren (e) seien genannt:
α-(7-(3,5-Dichlor-pyridin-2-yl-oxy)-, α-(7-(2,6-Dichlor-4-trifluormethyl-phenoxy)-, α-(7-(2-Chlor-6-fluor-4-trifluormethyl-phenoxy)-, α-(7-(2,3,6-Trichlor-4-trifluormethyl-phenoxy)- und α-(7-(2,6-Dichlor-3-fluor-4-trifluormethyl-phenoxy)-naphthalin-2-yl-oxy)-propionsäure-chlorid;
β-(7-(3,5-Dichlor-pyridin-2-yl-oxy)-, β-(7-(2,6-Dichlor-4-trifluormethyl-phenoxy)-, β-(7-(2-Chlor-6-fluor-4-trifluormethyl-phenoxy)-, β-(7-(2,3,6-Trichlor-4-trifluormethyl-phenoxy)- und β-(7-(2,6-Dichlor-3-fluor-4-trifluormethyl-phenoxy)-naphthalin-2-yl-oxy)-propionsäure-chlorid;
(7(3,5-Dichlor-pyridin-2-yl-oxy)-, (7-(2,6-Dichlor-4-trifluormethyl-phenoxy)-, (7-(2-Chlor-6-fluor-4-trifluormethyl-phenoxy)-, (7-(2,3,6-Trichlor-4-trifluormethyl-phenoxy)- und (7-(2,6-Dichlor-3-fluor-4-trifluormethyl-phenoxy)-naphthalin-2-yl-oxy)-essigsäure-chlorid.

Die oben beschriebenen Ausgangsstoffe der Formel (I) für Verfahren (e) sind erfindungsgemäße, neue Verbindungen; sie können nach den erfindungsgemäßen Verfahren (d) hergestellt werden.

Die beim erfindungsgemäßen Verfahren (e) als Ausgangsstoffe einzusetzenden Verbindungen sind durch die Formel (VI) allgemein definiert. In Formel (VI) hat Y vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurden.

Als Beispiele für die Ausgangsstoffe der Formel (VI) seien genannt:
Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxypropanol, 2-Benzylthio-ethanol, Hydroxymethanphosphonsäure-diethylester und -dimethylester, 1-Hydroxy-ethan-phosphonsäure-dimethylester und -diethylester, 1-Hydroxy-1-phenyl-methanphosphonsäuredimethylester und -diethylester, Acetonoxim, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (f) als Ausgangsstoffe zu verwendenden Verbindungen sind durch die Formel (I) mit der Maßgabe, daß Z für die Gruppierung -CO-Y steht, worin Y für Hydroxy steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (f) seien genannt:
α-(7-(3,5-Dichlor-pyridin-2-yl-oxy)-, α-(7-(2,6-Dichlor-4-trifluormethyl-phenoxy)-, α-(7-(2-Chlor-6-fluor-4-trifluormethyl-phenoxy)-, α-(7-(2,3,6-Trichlor-4-trifluormethyl-phenoxy)- und α-(7-(2,6-Dichlor-3-fluor-4-trifluormethyl-phenoxy)-naphthalin-2-yl-oxy)-propionsäure;
β-(7-(3,5-Dichlor-pyridin-2-yl-oxy)-, β-(7-(2,6-Dichlor-4-trifluormethyl-phenoxy)-, β-(7-(2-Chlor-6-fluor-4-trifluormethyl-phenoxy)-, β-(7-(2,3,6-Trichlor-4-trifluormethyl-phenoxy)- und β-(7-(2,6-Dichlor-3-fluor-4-trifluormethyl-phenoxy)-naphthalin-2-yl-oxy)-propionsäure;
(7-(3,5-Dichlor-pyridin-2-yl-oxy)-, (7-(2,6-Dichlor-4-trifluormethyl-phenoxy)-, (7-(2-Chlor-6-fluor-4-trifluormethyl-phenoxy)-, (7-(2,3,6-Trichlor-4-trifluormethyl-phenoxy)- und (7-(2,6-Dichlor-3-fluor-4-trifluormethyl-phenoxy)-naphthalin-2-yl-oxy)-essigsäure.

Die oben beschriebenen Ausgangsstoffe der Formel (I) für Verfahren (f) sind erfindungsgemäße, neue Verbindungen; sie können nach dem erfindungsgemäßen Verfahren (c) hergestellt werden.

Die beim erfindungsgemäßen Verfahren (f) weiter als Ausgangsstoffe einzusetzenden Verbindungen sind durch die Formel (VI) allgemein definiert. In Formel (VII) stehen vorzugsweise $R^{6-1}$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl und $Z^3$ für Chlor, Brom oder Iod.

Insbesondere stehen in Formel (VII) $R^{6-1}$ für Trimethylsilylmethyl und $Z^3$ für Chlor.

Trimethylsilylmethylchlorid wird als Ausgangsverbindung der Formel (VII) für Verfahren (f) besonders bevorzugt.

Die Ausgangstoffe der Formel (VII) sind bekannte Synthesechemikalien.

Verfahren (f) wird vorzugsweise unter Verwendung eines Vedünnungsmittels durchgeführt. Es kommen vor allem diejenigen Verdünnungsmittel in Betracht, die bereits bei der Beschreibung des erfindungsgemäßen Verfahrens (a) genannt wurden. Aceton, Acetonitril und Dimethylformamid werden besonders bevorzugt.

Verfahren (f) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden. 1,8-Diazabicyclo-[5.4.0]-undec-7-en (DBU) wird besonders bevorzugt.

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man je Mol Ausgangsverbindung der Formel (I) im allgemeinen zwischen 1 und 3 Mol, vorzugsweise zwischen 1,1 und 2,5 Mol Ausgangsverbindung der Formel (VII) ein.

Umsetzung und Aufarbeitung können wie oben für Verfahren (a) beschrieben durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Mengen ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung dikotyler Unkräuter, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (Amethydione) oder N-(2-Benzthiazolyl)-N,N′-dimethyl-harnstoff (Metabenzthiazuron) zur Unkrautbekämpfung in Getreide; 4-Amino-3-methyl-6-phenyl-1,2,4-triazin-5(4H)-on (Metamitron) zur Unkrautbekämpfung in Zuckerrüben und 4-Amino-6-(1,1-dimethylethyl)-3-methylthio-1,2,4-triazin-5(4H)-on (Metribuzin) zur Unkrautbekämpfung in Sojabohnen, in Frage, ferner auch 2,4-Dichlorphenoxyessigsäure (2,4-D); 2,4-Dichlorphenoxypropionsäure (2,4-DP); 4-(2,4-Dichlorphenoxy)-buttersäure (2,4-DB); Methyl-5-(2,4-dichlorphenoxy)-2-nitrobenzoat (Bifenox); 3,5-Dibrom-4-hydroxy-benzonitril (Bromoxynil); 2-Chlor-N-{[(4-methoxy-6-methyl-1,3,5-triazin-2-yl)-amino]-carbonyl}-benzolsulfonamid (Chlorsulfuron); N,N-Dimethyl-N′-(3-chlor-4-methylphenyl)-harnstoff (Chlortoluron); 2-[4-(2,4-Dichlorphenoxy)-phenoxy]-propionsäure, deren Methyl- oder deren Ethylester; 3,6-Dichlor-2-pyridincarbonsäure (Clopyralid); 2-{4-[(6-Chlor-2-benzoxazolyl)-oxy]-phenoxy}-propansäure, deren Methyl- oder deren Ethylester (Fenoxaprop); 2-[4-(3,5-Dichlorpyrid-2-yloxy)-phenoxy]-propionsäure-(trimethylsilylmethylester); Methyl-2-[4,5-dihydro-4-methyl-4-(1-methylethyl)-5-oxo-1H-imidazol-2-yl]-4(5)-methylbenzoat (Imazamethabenz); 2-[4,5-Dihydro-4-methyl-4-isopropyl-5-oxo-(1H)-imidazol-2-yl]-5-ethyl-pyridin-3-carbonsäure (Imazethapyr); 3,5-Diiod-4-hydroxybenzonitril (Ioxynil); N,N-Dimethyl-N′-(4-isopropylphenyl)-harnstoff (Isoproturon); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (Mefenazet); 2-{[[((4-Methoxy-6-methyl-1,3,5-triazin-2-yl)-amino)-carbonyl]-amino]-sulfonyl}-benzoesäure oder deren Methylester (Metsulfuron); N-(1-Ethylpropyl)-3,4-dimethyl-2,6-dinitroanilin (Pendimethalin); 3-[[[[(4-Methoxy-6-methyl-1,3,5-triazin-2-yl}-amino]-carbonyl]-amino]-sulfonyl]-thiophen-2-carbonsäure-methylester (Thiameturon) sowie 3,5,6-Trichlor-2-pyridyloxyessigsäure (Triclopyr). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

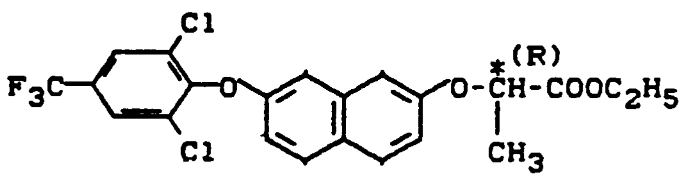

Eine Mischung aus 16,8 g (0,045 Mol) 7-(2,6-Dichlor-4-trifluormethyl-phenoxy)-2-naphthol, 13,6 g (0,050 Mol) (S)-α-(4-Methyl-phenylsulfonyloxy)-propionsäure-ethylester, 6,3 g Kaliumcarbonat und 150 ml Acetonitril wird 20 Stunden unter Rückfluß zum Sieden erhitzt. Nach dem Abkühlen auf ca. 20 °C wird das Reaktionsgemisch mit Wasser auf etwa das doppelte Volumen verdünnt, mit 1N-Salzsäure angesäuert und mit Toluol geschüttelt. Die organische Phase wird abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 17,6 g (83 % der Theorie) (R)-α-(7-(2,6-Dichlor-4-trifluormethyl-phenoxy)-naphthalin-2-yl-oxy)-propionsäure-ethylester als amorphen Rückstand

$$(^{1}\text{H-NMR } (CDCl_3)\text{: } \delta_{C\underline{H}CH_3} = 4{,}8 \text{ ppm (q)}).$$

Analog Beispiel 1 und entsprechend der allgemeinen Beschreibung der erfindungsgemäßen Herstellungsverfahren können die in der nachstehenden Tabelle 1 aufgeführten Verbindungen der Formel (I) hergestellt werden:

**Tabelle 1**: Beispiele für die Verbindungen der Formel (I)

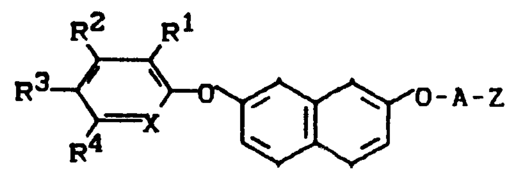

(I)

| Beisp.-Nr. | $R^1$ | $R^2$ | $R^3$ | $R^4$ | X | A | Z | Physikalische Daten |
|---|---|---|---|---|---|---|---|---|
| 2 | Cl | Cl | $CF_3$ | H | C-Cl | -CH-<br>$CH_3$ | $COOCH_2P(O)(OCH_3)_2$ | |
| 3 | Cl | H | Cl | H | N | -CH-<br>$CH_3$ | $COOC_2H_5$ | $n_D^{20}$ = 1,6080 |
| 4 | Cl | H | $CF_3$ | H | C-F | -CH-<br>$CH_3$ | $COOC_2H_5$ | $n_D^{20}$ = 1,5638 |
| 5 | Cl | Cl | $CF_3$ | H | C-Cl | -CH-<br>$CH_3$ | $COOC_4H_9$ | |
| 6 | Cl | F | $CF_3$ | H | C-Cl | -CH-<br>$CH_3$ | $COOCH(CH_3)_2$ | |
| 7 | Cl | H | $CF_3$ | H | C-Cl | $-CH_2-$ | $COOC_4H_9$ | |
| 8 | Cl | H | $CF_3$ | H | C-Cl | $-CH_2CH_2-$ | $COOCH_3$ | |

**Tabelle 1** - Fortsetzung

| Beisp.-Nr. | $R^1$ | $R^2$ | $R^3$ | $R^4$ | X | A | Z | Physikalische Daten |
|---|---|---|---|---|---|---|---|---|
| 9 | Cl | H | $CF_3$ | H | C-Cl | $-CH(CH_3)-$ | COOH | Fp: 138° C |
| 10 | Cl | H | Cl | H | N | $-CH_2-$ | COOH | |
| 11 | Cl | H | Cl | H | N | $-CH(CH_3)-$ | COOH | |
| 12 | Cl | H | $CF_3$ | H | C-Cl | $-CH_2-$ | COOH | |
| 13 | Cl | H | Cl | H | N | $-CH(CH_3)-$ | COCl | |
| 14 | Cl | H | $CF_3$ | H | C-Cl | $-CH(CH_3)-$ | COCl | |
| 15 | Cl | H | $CF_3$ | H | C-Cl | $-CH(CH_3)-$ | $COO-CH_2-COOC_4H_9$ | |
| 16 | Cl | H | $CF_3$ | H | C-Cl | $-CH(CH_3)-$ | $COOCH(CH_3)COOC_2H_5$ | |

**Tabelle 1** - Fortsetzung

| Beisp.-Nr. | $R^1$ | $R^2$ | $R^3$ | $R^4$ | X | A | Z | Physikalische Daten |
|---|---|---|---|---|---|---|---|---|
| 17 | Cl | H | $CF_3$ | H | C-Cl | $-CH_2-$ | $COOCHCOOC_2H_5$<br>\|<br>$CH_3$ | |
| 18 | Cl | Cl | $CF_3$ | H | C-Cl | $-CH_2-$ | $COSC_2H_5$ | |
| 19 | Cl | F | $CF_3$ | H | C-Cl | $-CH-$<br>\|<br>$CH_3$ | $COSCH_2COOCH_3$ | |
| 20 | Cl | H | Cl | H | N | $-CH_2CH_2-$ | $CONHC_2H_5$ | |
| 21 | Cl | H | $CF_3$ | H | C-Cl | $-CH_2-$ | COCl | |
| 22 | Cl | H | $CF_3$ | H | C-Cl | $-CH_2CH_2-$ | COCl | |
| 23 | Cl | H | $CF_3$ | H | C-Cl | $-CH_2CH_2-$ | COOH | |
| 24 | Cl | H | Cl | H | N | $-CH_2CH_2-$ | COOH | |
| 25 | Cl | H | Cl | H | N | $-CH-$<br>\|<br>$CH_3$ | $COOCH_2CH_2OCH_3$ | |
| 26 | Cl | H | $CF_3$ | H | C-Cl | $-CH-$<br>\|<br>$CH_3$ | $COOCH_2CH_2SC_2H_5$ | |

**Tabelle 1 - Fortsetzung**

| Beisp.-Nr. | $R^1$ | $R^2$ | $R^3$ | $R^4$ | X | A | Z | Physikalische Daten |
|---|---|---|---|---|---|---|---|---|
| 27 | Cl | H | Cl | H | N | $-CH_2-$ | $COOCH_2-\overset{\overset{O}{\|}}{P}(OC_2H_5)_2$ | |
| 28 | Cl | H | $CF_3$ | H | C-Cl | $-CH_2-$ | $COO\underset{C_6H_5}{CH}-\overset{\overset{O}{\|}}{P}(OCH_3)_2$ | |
| 29 | Cl | H | $CF_3$ | H | C-Cl | $-CH_2CH_2-$ | $CONHCH(CH_3)_2$ | |
| 30 | Cl | Cl | $CF_3$ | H | C-Cl | $-CH_2-$ | $CON(C_2H_5)_2$ | |
| 31 | Cl | F | $CF_3$ | H | C-Cl | $-\underset{CH_3}{CH}-$ | $COOCH_2CH_2OCH_2-$ 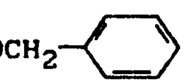 | |
| 32 | Cl | H | $CF_3$ | H | C-F | $-\underset{CH_3}{CH}-$ | $COOCH_2$ 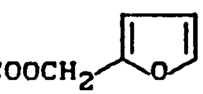 | |
| 33 | Cl | H | Cl | H | N | $-CH_2-$ | $CONHOCH_3$ | |

<u>Tabelle 1</u> - Fortsetzung

| Beisp.-Nr. | $R^1$ | $R^2$ | $R^3$ | $R^4$ | X | A | Z | Physikalische Daten |
|---|---|---|---|---|---|---|---|---|
| 34 | Cl | H | $CF_3$ | H | C-Cl | $-CH_2CH_2-$ | $CONHOC_2H_5$ | |
| 35 | Cl | Cl | $CF_3$ | H | C-Cl | $-CH-$<br>\|<br>$CH_3$ | $CONHCH_2COOC_2H_5$ | |
| 36 | Cl | H | $CF_3$ | H | C-Cl | $-CH_2-$ | $CONHNHSO_2CH_3$ | |
| 37 | Cl | H | Cl | H | N | $-CH_2-$ | $COOCH_2Si(CH_3)_3$ | |
| 38 | Cl | H | $CF_3$ | H | C-Cl | $-CH-$<br>\|<br>$CH_3$ | $COO(CH_2)_3OCH_2-$ 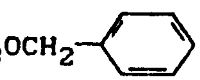 | |
| 39 | Cl | H | $CF_3$ | H | C-Cl | $-CH_2-$ | CN | Fp: 163 °C |
| 40 | Cl | H | $CF_3$ | H | C-Cl | $-CH_2CH_2-$ | CN | |
| 41 | Cl | H | Cl | H | N | $-CH_2-$ | CN | |
| 42 | Cl | Cl | $CF_3$ | H | C-Cl | $-CH_2-$ | CN | |
| 43 | Cl | F | $CF_3$ | H | C-Cl | $-CH_2-$ | CN | |
| 44 | Cl | H | $CF_3$ | H | C-F | $-CH_2-$ | CN | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 45 | Cl | Cl | $CF_3$ | H | C-Cl | -CH-<br>\|<br>$CH_3$ | $COOC_2H_5$ | (amorph) |
| 46 | Cl | H | $CF_3$ | H | C-Cl | -CH-(R)<br>\|<br>$CH_3$ | $COOCH_3$ | Fp: 89° C |
| 47 | Cl | H | $CF_3$ | H | C-Cl | $-CH_2CH_2-$ | $COOC_2H_5$ | |
| 48 | F | F | $CF_3$ | F | C-F | -CH-<br>\|<br>$CH_3$ | $COOC_2H_5$ | |
| 49 | Cl | H | $CF_3$ | H | C-F | -CH-<br>\|<br>$CH_3$ | COOH | |
| 50 | Cl | H | $CF_3$ | H | C-F | -CH-<br>\|<br>$CH_3$ | $COOCH_2Si(CH_3)_3$ | |
| 51 | Cl | H | $CF_3$ | H | C-F | -CH-<br>\|<br>$CH_3$ | O<br>‖<br>$COOCH_2P(OC_2H_5)_2$ | |
| 52 | Cl | H | $CF_3$ | H | C-Cl | -CH-<br>\|<br>$CH_3$ | $COOCH_2Si(CH_3)_3$ | |

**Tabelle 1** - Fortsetzung

| Beisp.-Nr. | $R^1$ | $R^2$ | $R^3$ | $R^4$ | X | A | Z | Physikalische Daten |
|---|---|---|---|---|---|---|---|---|
| 53 | Cl | H | $CF_3$ | H | C-Cl | $-CH(CH_3)-$ | $COOCH_2P(=O)(OC_2H_5)_2$ | |
| 54 | Cl | H | $CF_3$ | H | C-Cl | $-CH(CH_3)-$ | $COOCH_2CH_2OC_2H_5$ | |
| 55 | Cl | H | $CF_3$ | H | C-Cl | $-CH_2-$ | $CO-NH_2$ | |
| 56 | Cl | H | $CF_3$ | H | C-Cl | $-CH_2-$ | $CO-N(C_3H_7)_2$ | Fp: 109° C |
| 57 | Cl | H | $CF_3$ | H | C-Cl | $-CH_2-$ | $CO-N(CH_2CH=CH_2)_2$ | |
| 58 | Cl | H | $CF_3$ | H | C-Cl | $(-CH_2)_3-$ | $COOC_2H_5$ | Fp: 75° C |
| 59 | Cl | H | $CF_3$ | H | C-Cl | $-CH(C_2H_5)-$ | $COOC_2H_5$ | Fp: 118° C |
| 60 | Cl | H | $CF_3$ | H | C-Cl | $-CH(CH_3)-$ | $COOCH_2$ 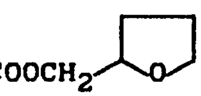 | |
| 61 | Cl | H | $CF_3$ | H | C-Cl | $-CH(CH_3)-$ | $COOCH(CH_3)P(=O)(OCH_3)_2$ | |

**Tabelle 1** Fortsetzung

| Beispiel Nr. | $R^1$ | $R^2$ | $R^3$ | $R^4$ | X | A | Z | Physikalische Daten |
|---|---|---|---|---|---|---|---|---|
| 62 | Cl | H | $CF_3$ | H | C-Cl | $-CH_2-$ | $COOCH_3$ | Fp: 98° C |
| 63 | Cl | H | $CF_3$ | H | C-Cl | $-CH_2-$ | 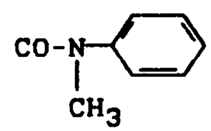 | Fp: 108° C |
| 64 | Cl | H | $CF_3$ | H | C-Cl | $-CH_2-$ | $COOC_2H_5$ | Fp: 76° C |
| 65 | Cl | H | $SO_2CF_3$ | H | CH | -CH-(R)<br>$CH_3$ | $COOC_2H_5$ | |
| 66 | Cl | H | $CF_3$ | H | C-Cl | -CH-(R/S)<br>$CH_3$ | $COOC_2H_5$ | Fp: 74° C |
| 67 | Cl | H | $CF_3$ | F | C-F | -CH-<br>$CH_3$ | $COOC_2H_5$ | |
| 68 | $CF_3$ | H | $CF_3$ | H | CH | -CH-<br>$CH_3$ | $COOC_2H_5$ | |

**Tabelle 1 - Fortsetzung**

| Beisp.-Nr. | $R^1$ | $R^2$ | $R^3$ | $R^4$ | X | A | Z | Physikalische Daten |
|---|---|---|---|---|---|---|---|---|
| 69 | Cl | H | $CF_3$ | H | C-Cl | $-CH-$(S)<br>\|<br>$CH_3$ | $COOCH_3$ | |
| 70 | $CF_3$ | H | $CF_3$ | H | C-Cl | $-CH-$(R)<br>\|<br>$CH_3$ | $COOC_2H_5$ | Fp: 92° C |
| 71 | Cl | H | $SO_2CF_3$ | H | C-Cl | $-CH-$(R)<br>\|<br>$CH_3$ | $COOC_2H_5$ | |
| 72 | Cl | H | $CF_3$ | H | C-F | $-CH-$<br>\|<br>$CH_3$ | $COOCH_3$ | |
| 73 | Cl | H | $CF_3$ | H | C-F | $-CH_2-$ | $COOCH_3$ | Fp: 93° C |
| 74 | Cl | H | $CF_3$ | H | C-F | $-CH-$<br>\|<br>$C_2H_5$ | $COOC_2H_5$ | |
| 75 | $CF_3$ | H | $CF_3$ | H | C-F | $-CH-$<br>\|<br>$CH_3$ | $COOC_2H_5$ | |
| 76 | Cl | H | $SO_2CF_3$ | H | C-F | $-CH_2-$ | $COOC_2H_5$ | zähes Öl |

Ausgangsstoffe der Formel (II)

Beispiel (II-1)

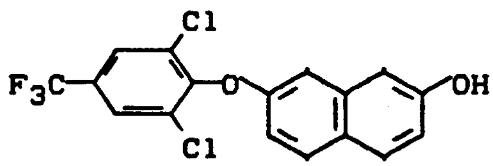

12,5 g (0,05 Mol) 3,4,5-Trichlor-benzotrifluorid werden zu einer auf 60 °C erwärmten Mischung aus 8,0 g (0,05 Mol) 2,7-Dihydroxy-naphthalin, 4,2 g (0,075 Mol) Kaliumhydroxid-Pulver und 100 ml Dimethylsulfoxid langsam unter Rühren gegeben und das Reaktionsgemisch wird dann noch ca. 3 Stunden bei 60 °C gerührt. Nach Abkühlen auf ca. 20 °C wird mit Wasser und Methylenchlorid verdünnt und filtriert. Vom Filtrat wird die organische Phase abgetrennt, mit Wasser gewaschen, mit Natriumsulfat getrocknet und filtriert. Vom Filtrat wird das Lösungsmittel im Wasserstrahlvakuum abdestilliert, der Rückstand mit Petrolether verrührt und das hierbei kristallin angefallene Produkt durch Absaugen isoliert.

Man erhält 4,9 g (26 % der Theorie) 7-(2,6-Dichlor-4-trifluormethyl-phenoxy)-2-naphthol vom Schmelzpunkt 98 °C.

Analog Beispiel (II-1) können die in der nachstehenden Tabelle 2 aufgeführten Ausgangsstoffe der Formel (II) hergestellt werden.

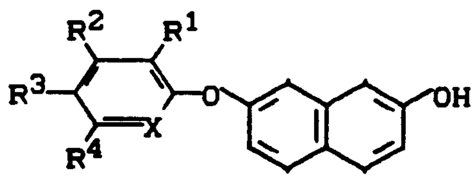

(II)

**Tabelle 2: Beispiele für die Ausgangsstoffe der Formel (II)**

| Beisp.-Nr. | $R^1$ | $R^2$ | $R^3$ | $R^4$ | X | Physikalische Daten |
|---|---|---|---|---|---|---|
| II-2 | Cl | Cl | $CF_3$ | H | C-Cl | |
| II-3 | Cl | F | $CF_3$ | H | C-Cl | |
| II-4 | Cl | H | $CF_3$ | H | C-F | Fp: 91°C |
| II-5 | Cl | H | Cl | H | N | Fp: 152 °C |

Anwendungsbeispiele

In den folgenden Anwendungsbeispielen wird die nachstehend aufgeführte Verbindung als Vergleichssubstanz herangezogen:

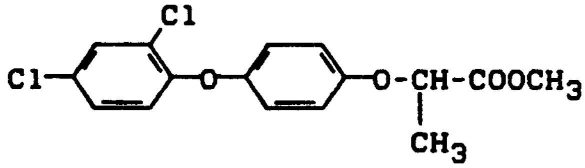

(A)

α-(4-(2,4-Dichlor-phenoxy)-phenoxy)-propionsäure-methylester (bekannt aus DE-OS 22 23 894/Verbindung 86).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

In diesem Test zeigten beispielsweise die Verbindungen gemäß Herstellungsbeispiel (1) und (4) bei guter Selektivität in Weizen erheblich stärkere Wirkung gegen Problemunkräuter, wie z. B. Amaranthus, Galium, Galinsoga, Ipomoea, Veronica und Viola, als die Vergleichssubstanz (A).

## Patentansprüche

**1.** (7-(Hetero)Aryloxy-naphthalin-2-yl-oxy)-alkancarbonsäure-Derivate der Formel (I),

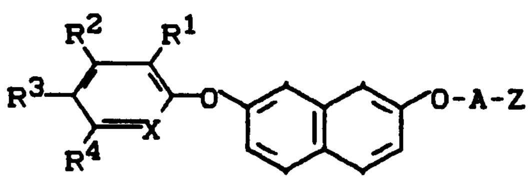

(I)

in welcher

- $R^1$ für Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- X für Stickstoff oder die Gruppierung C-$R^5$ steht, worin
- $R^5$ für Wasserstoff oder Halogen steht,

- mit der Maßgabe, daß wenigstens einer der Substituenten $R^2$ oder $R^4$ von Wasserstoff verschieden ist, wenn $R^1$ für Chlor oder Cyano und gleichzeitig $R^3$ für Trifluormethyl sowie X für CH steht und daß $R^3$ von Trifluormethyl verschieden ist, wenn X für Stickstoff steht -

- A für gegebenenfalls verzweigtes Alkandiyl steht und
- Z für Cyano oder die Gruppierung -CO-Y steht, worin
- Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, N-Alkylarylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
- $R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Aralkoxyalkyl, Trialkylsilylalkyl, Arylthioalkyl, Aralkylthioalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl,

Alkylidenamino oder für ein Ammonium-, Alkylammonium-, Alkalimetall-oder Erdalkalimetall-äquivalent steht
oder für die Gruppierung

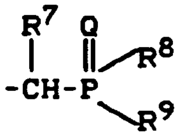

steht,
worin

$R^7$ für Wasserstoff, Alkyl, Aryl, Furyl, Thienyl oder Pyridyl steht,
$R^8$ für Alkyl oder Alkoxy steht,
$R^9$ für Alkoxy steht und
Q für Sauerstoff oder Schwefel steht,
oder
$R^6$ für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht.

**2.** (7-(Hetero)Aryloxy-naphthalin-2-yl-oxy)-alkancarbonsäure-Derivate der Formel (I), gemäß Anspruch 1, in welcher

$R^1$ für Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht,
X für Stickstoff oder die Gruppierung $C-R^5$ steht, worin
$R^5$ für Wasserstoff, Fluor, Chlor oder Brom steht,

- mit der Maßgabe, daß wenigstens einer der Substituenten $R^2$ oder $R^4$ von Wasserstoff verschieden ist, wenn $R^1$ für Chlor oder Cyano und gleichzeitig $R^3$ für Trifluormethyl sowie X für CH steht und daß $R^3$ von Trifluormethyl verschieden ist, wenn X für Stickstoff steht -

A für gegebenenfalls verzweigtes $C_1$-$C_4$-Alkandiyl steht und
Z für Cyano oder die Gruppierung -CO-Y steht, worin
Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di($C_1$-$C_4$-alkyl)-amino, Di-($C_3$-$C_4$-alkenyl)-amino, N-($C_1$-$C_4$-alkyl)-phenylamino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung $-O-R^6$ steht, worin
$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Tri-($C_1$-$C_2$-alkyl)-silyl-$C_1$-$C_2$-alkyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- oder Calcium-äquivalent steht, oder für
die Gruppierung

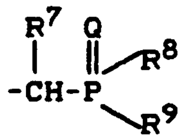

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

**3.** Verfahren zur Herstellung von (7-(Hetero)Aryloxynaphthalin-2-yl-oxy)-alkancarbonsäure-Derivaten der Formel (I),

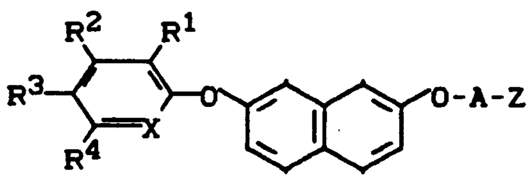

(I)

in welcher

$R^1$ für Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

X für Stickstoff oder die Gruppierung C-$R^5$ steht, worin

$R^5$ für Wasserstoff oder Halogen steht,

- mit der Maßgabe, daß wenigstens einer der Substituenten $R^2$ oder $R^4$ von Wasserstoff verschieden ist, wenn $R^1$ für Chlor oder Cyano und gleichzeitig $R^3$ für Trifluormethyl sowie X für CH steht und daß $R^3$ von Trifluormethyl verschieden ist, wenn X für Stickstoff steht -

A für gegebenenfalls verzweigtes Alkandiyl steht und

Z für Cyano oder die Gruppierung -CO-Y steht, worin

Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, N-Alkylarylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Aralkoxyalkyl, Trialkylsilylalkyl, Arylthioalkyl, Aralkylthioalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetalläquivalent steht oder für die Gruppierung

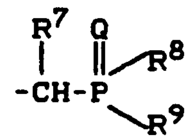

steht,
worin

$R^7$ für Wasserstoff, Alkyl, Aryl, Furyl, Thienyl oder Pyridyl steht,
$R^8$ für Alkyl oder Alkoxy steht,
$R^9$ für Alkoxy steht und
Q für Sauerstoff oder Schwefel steht,
oder
$R^6$ für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetra-hydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht,

dadurch gekennzeichnet, daß man

(a) 7-(Hetero)Aryloxy-2-naphthole der allgemeinen Formel (II)

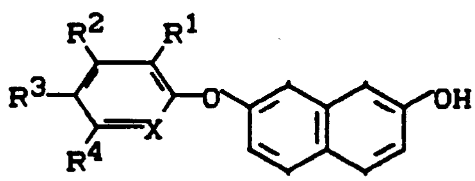

(II)

in welcher
$R^1$, $R^2$, $R^3$, $R^4$ und X die oben angegebenen Bedeutungen haben,
mit Carbonsäurederivaten der allgemeinen Formel (III)

$Z^1$ - A - Z (III)

in welcher
A und Z die oben angegebenen Bedeutungen haben und
$Z^1$ für eine nucleophile Abgangsgruppe steht,
gegebenenfalls in Gegenwart eines Säureakzeptors und gegebenenfalls in Gegenwart eines Verdünnungsmittels umsetzt, oder daß man

(b) Halogen-(hetero)aryl-Verbindungen der allgemeinen Formel (IV)

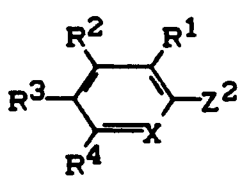

(IV)

in welcher
$R^1$, $R^2$, $R^3$, $R^4$ und X die oben angegebenen Bedeutungen haben und
$Z^2$ für Halogen steht,
mit (7-Hydroxy-naphthalin-2-yl-oxy)-alkancarbonsäure-Derivaten der allgemeinen Formel (V)

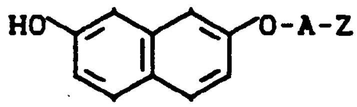

(V)

in welcher

A und Z die oben angegebenen Bedeutungen haben,

gegebenenfalls in Gegenwart eines Säureakzeptors und gegebenenfalls in Gegenwart eines Verdünnungsmittels umsetzt, oder daß man

(c) für den Fall, daß in der Formel (I) Z für die Gruppierung -CO-Y steht, worin Y für Hydroxy steht und A, $R^1$, $R^2$, $R^3$, $R^4$ und X die oben angegebenen Bedeutungen haben, Verbindungen der Formel (I), in welcher Z für Cyano oder für die Gruppierung -CO-Y steht, worin Y für Methoxy oder Ethoxy steht und A, $R^1$, $R^2$, $R^3$, $R^4$ und Y die oben angegebenen Bedeutungen haben, mit einem Alkalihydroxid in Gegenwart eines organischen Lösungsmittels umsetzt und dann, gegebenenfalls nach Einengen, mit einer Mineralsäure ansäuert, oder daß man

(d) für den Fall, daß in der Formel (I) Z für die Gruppierung -CO-Y steht, worin Y für Halogen steht und A, $R^1$, $R^2$, $R^3$, $R^4$ und X die oben angegebenen Bedeutungen haben, Verbindungen der allgemeinen Formel (I), in welcher Z für die Gruppierung -CO-Y steht, worin Y für Hydroxy steht und A, $R^1$, $R^2$, $R^3$, $R^4$ und X die oben angegebenen Bedeutungen haben, mit einem Halogenierungsmittel gegebenenfalls in Gegenwart eines Verdünnungsmittels umsetzt, oder daß man

(e) für den Fall, daß Z in der Formel (I) für die Gruppierung -CO-Y steht, worin Y mit Ausnahme von Halogen die oben angegebene Bedeutung hat und A, $R^1$, $R^2$, $R^3$, $R^4$ und X die oben angegebenen Bedeutungen haben,

Verbindungen der allgemeinen Formel (I), in welcher Z für Cyano oder die Gruppierung -CO-Y steht, worin Y für Halogen steht und A, $R^1$, $R^2$, $R^3$, $R^4$ und X die oben angegebenen Bedeutungen haben, mit Verbindungen der allgemeinen Formel (VI)

$$H - Y \qquad (VI)$$

in welcher

Y mit Ausnahme von Halogen die oben angegebene Bedeutung hat,

gegebenenfalls in Gegenwart einer Säure bzw. eines Säureakzeptors und gegebenenfalls in Gegenwart eines Verdünnungsmittels umsetzt, oder daß man

(f) für den Fall, daß Z in der Formel (I) für die Gruppierung -CO-Y steht, worin Y für die Gruppierung $-O-R^6$ steht, worin $R^6$ mit Ausnahme von Ammonium, Alkylammonium, Alkalimetall und Erdalkalimetall die oben angegebene Bedeutung hat und A, $R^1$, $R^2$, $R^3$, $R^4$ und X die oben angegebenen Bedeutungen haben, Verbindungen der allgemeinen Formel (I), in welcher Z für die Gruppierung -CO-Y steht, worin Y für Hydroxy steht und A, $R^1$, $R^2$, $R^3$, $R^4$ und Y die oben angegebenen Bedeutungen haben,

mit Verbindungen der allgemeinen Formel (VII)

$$Z^3 - R^{6-1} \qquad (VII)$$

in welcher

$R^{6-1}$ mit Ausnahme von Ammonium, Alkylammonium, Alkalimetall und Erdalkalimetall die oben für $R^6$ angegebene Bedeutung hat und

$Z^3$ für Halogen steht,

gegebenenfalls in Gegenwart eines Säureakzeptors und gegebenenfalls in Gegenwart eines Verdünnungsmittels umsetzt.

4. Herbizide Mittel, gekennzeichnet durch einen Gehalt an mindestens einem (7-(Hetero)Aryloxy-naphthalin-2-yl-oxy)-alkancarbonsäure-Derivat der Formel (I) gemäß den Ansprüchen 1 bis 3.

5. Verfahren zur Bekämpfung von Unkräutern, dadurch gekennzeichnet, daß man (7-(Hetero)Aryloxy-naphthalin-2-yl-oxy)-alkancarbonsäure-Derivate der Formel (I) gemäß den Ansprüchen 1 bis 3 auf die Unkräuter und/oder ihren Lebensraum einwirken läßt.

**6.** Verwendung von (7-(Hetero)Aryloxy-naphthalin-2-yl-oxy)-alkancarbonsäure-Derivaten der Formel (I) gemäß den Ansprüchen 1 bis 3 zur Bekämpfung von Unkräutern.

**7.** Verfahren zur Herstellung von herbiziden Mitteln, dadurch gekennzeichnet, daß man (7-(Hetero)Aryloxy-naphthalin-2-yl-oxy)-alkancarbonsäure-Derivate der Formel (I) gemäß den Ansprüchen 1 bis 3 mit Streckmitteln und/oder oberflächenaktiven Substanzen vermischt.

**8.** Die Verbindung der Formel

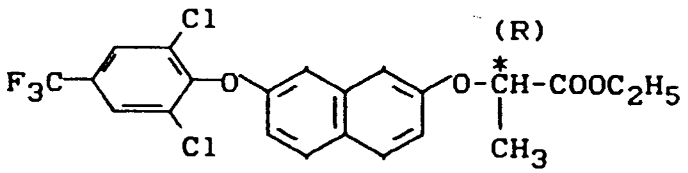

gemäß Anspruch 1.

**9.** 7-(Hetero)Aryloxy-2-naphthole der Formel (IIa),

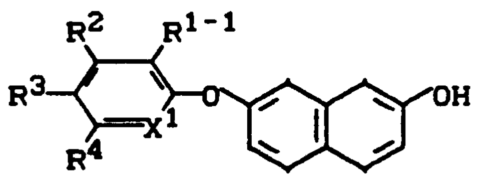

(IIa)

in welcher

- $R^{1-1}$ für Halogen oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- $X^1$ für Stickstoff oder die Gruppierung C-$R^{5-1}$ steht, worin
- $R^{5-1}$ für Halogen steht.

**10.** Verfahren zur Herstellung von 7-(Hetero)-Aryloxy-2-naphtholen der Formel (IIa),

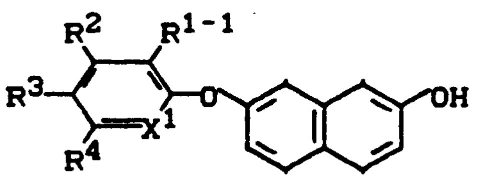

(IIa)

in welcher

- $R^{1-1}$ für Halogen oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- $X^1$ für Stickstoff oder die Gruppierung C-$R^{5-1}$ steht, worin
- $R^{5-1}$ für Halogen steht,

dadurch gekennzeichnet, daß man Halogen(hetero)-aryl-Verbindungen der Formel (IVa),

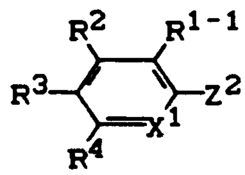

(IVa)

in welcher

$R^{1-1}$, $R^2$, $R^3$, $R^4$ und $X^1$ die oben angegebenen Bedeutungen haben und

$Z^2$ für Halogen steht,

mit 2,7-Dihydroxynaphthalin in Gegenwart eines Säureakzeptors und in Gegenwart eines Verdünnungsmittels umsetzt.

## Claims

1. (7-(Hetero)aryloxynaphthalen-2-yl-oxy)-alkanecarboxylic acid derivatives of the formula (I)

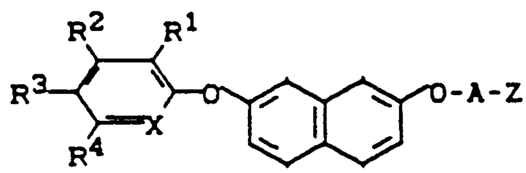

(I)

in which

$R^1$ represents halogen, cyano or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen,

X represents nitrogen or the grouping C-$R^5$, where

$R^5$ represents hydrogen or halogen,

- with the proviso that at least one of the substituents $R^2$ or $R^4$ is different from hydrogen if $R^1$ represents chlorine or cyano and simultaneously $R^3$ represents trifluoromethyl and X represents CH, and that $R^3$ is different from trifluoromethyl if X represents nitrogen -

A represents optionally branched alkanediyl, and

Z represents cyano or the grouping -CO-Y, where

Y represents halogen, hydroxyl, amino, alkylamino, alkenylamino, alkinylamino, arylamino, aralkylamino, alkoxycarbonylalkylamino, cyanoamino, dialkylamino, dialkenylamino, N-alkylarylamino, alkylsulphonylamino, arylsulphonylamino, hydroxyamino, alkoxyamino, hydrazino, alkylsulphonylhydrazino, arylsulphonylhydrazino, alkylthio, arylthio, aralkylthio, alkoxycarbonylalkylthio or the grouping -O-$R^6$, where

$R^6$ represents an optionally halogen-substituted radical from the series comprising alkyl, alkenyl, alkinyl, alkoxyalkyl, alkylthioalkyl, alkylsulphinylalkyl, alkylsulphonylalkyl, aryloxyalkyl, aralkoxyalkyl, trialkylsilylalkyl, arylthioalkyl, aralkylthioalkyl, alkoxycarbonylalkyl, alkylaminocarbonylalkyl, arylaminocarbonylalkyl, N-alkyl-N-aryl-aminocarbonylalkyl, aralkyl, azolylalkyl and alkylideneamino, or an ammonium equivalent, alkylammonium equivalent, alkali metal equivalent or alkaline earth metal equivalent, or

the grouping

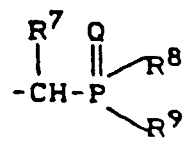

where

$R^7$ represents hydrogen, alkyl, aryl, furyl, thienyl or pyridyl,

$R^8$ represents alkyl or alkoxy,

$R^9$ represents alkoxy and

Q represents oxygen or sulphur,

or

$R^6$ represents the grouping $-(CH_2)_n-R^{10}$, where

$R^{10}$ represents an optionally halogen-substituted and/or alkyl-substituted heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl and pyrimidinyl, and

n represents the numbers 0, 1 or 2.

**2.** (7-(Hetero)aryloxynaphthalen-2-yl-oxy)alkanecarboxylic acid derivatives of the formula (I) according to Claim 1, in which

$R^1$ represents fluorine, chlorine, bromine, cyano or trifluoromethyl,

$R^2$ represents hydrogen, fluorine or chlorine,

$R^3$ represents fluorine, chlorine, bromine, trifluoromethyl, trifluoromethoxy, trifluoromethylthio or trifluoromethylsulphonyl,

$R^4$ represents hydrogen, fluorine or chlorine,

X represents nitrogen or the grouping $C-R^5$, in which

$R^5$ represents hydrogen, fluorine, chlorine or bromine,

- with the proviso that at least one of the substituents $R^2$ or $R^4$ is different from hydrogen if $R^1$ represents chlorine or cyano and simultaneously $R^3$ represents trifluoromethyl and X represents CH, and that $R^3$ is different from trifluoromethyl if X represents nitrogen -

A represents optionally branched $C_1$-$C_4$-alkanediyl and

Z represents cyano or the grouping -CO-Y, where

Y represents chlorine, hydroxyl, amino, $C_1$-$C_6$-alkylamino, $C_3$-$C_4$-alkenylamino, $C_3$-$C_4$-alkinylamino, phenylamino, benzylamino, $C_1$-$C_4$-alkoxycarbonyl-$C_1$-$C_2$-alkylamino, cyanoamino, di-($C_1$-$C_4$-alkyl)-amino, di-($C_3$-$C_4$-alkenyl)-amino, N-($C_1$-$C_4$-alkyl)-phenylamino, $C_1$-$C_4$-alkylsulphonylamino, phenylsulphonylamino, tolylsulphonylamino, hydroxyamino, $C_1$-$C_6$-alkoxyamino, hydrazino, $C_1$-$C_4$-alkylsulphonylhydrazino, phenylsulphonylhydrazino, tolylsulphonylhydrazino, $C_1$-$C_4$-alkylthio, phenylthio, benzylthio, $C_1$-$C_4$-alkoxy-carbonyl-$C_1$-$C_2$-alkylthio, or the grouping $-O-R^6$, where

$R^6$ represents an optionally fluorine-substituted and/or chlorine-substituted radical from the series comprising $C_1$-$C_6$-alkyl, $C_3$-$C_4$-alkenyl, $C_3$-$C_4$-alkinyl, $C_1$-$C_4$-alkoxy-$C_1$-$C_4$-alkyl, $C_1$-$C_4$-alkylthio-$C_1$-$C_4$-alkyl, $C_1$-$C_4$-alkylsulphinyl-$C_1$-$C_4$-alkyl, $C_1$-$C_4$-alkylsulphonyl-$C_1$-$C_4$-alkyl, phenoxy-$C_1$-$C_3$-alkyl, tri-($C_1$-$C_2$-alkyl)-silyl-$C_1$-$C_2$-alkyl, phenylthio-$C_1$-$C_3$-alkyl, benzyloxy-$C_1$-$C_3$-alkyl, benzylthio-$C_1$-$C_3$-alkyl, $C_1$-$C_4$-alkoxy-carbonyl-$C_1$-$C_2$-alkyl, $C_1$-$C_4$-alkylaminocarbonyl-$C_1$-$C_2$-alkyl, phenylaminocarbonyl-$C_1$-$C_4$-alkyl, N-($C_1$-$C_4$-alkyl)-N-phenylaminocarbonyl-$C_1$-$C_4$-alkyl, benzyl, pyrazolyl-$C_1$-$C_4$-alkyl, $C_2$-$C_4$-alkylideneamino or an ammonium equivalent, a $C_1$-$C_4$-alkylammonium equivalent, a sodium equivalent, a potassium equivalent or a calcium equivalent, or the grouping

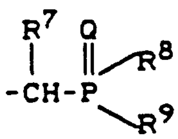

where

$R^7$ represents hydrogen, $C_1$-$C_4$-alkyl, phenyl, furyl, thienyl or pyridyl,

$R^8$ represents $C_1$-$C_4$-alkyl or $C_1$-$C_4$-alkoxy,

$R^9$ represents $C_1$-$C_4$-alkoxy and

Q represents oxygen or sulphur,

or

$R^6$ represents the grouping $-(CH_2)_n-R^{10}$, where

n represents the numbers 0, 1 or 2, and

$R^{10}$ represents a heterocyclic radical which is optionally substituted by fluorine, chlorine, bromine and/or $C_1$-$C_4$-alkyl, and which is from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl.

**3.** Process for the preparation of (7-(hetero)aryloxynaphthalen-2yl-oxy)-alkanecarboxylic acid derivatives of the formula (I)

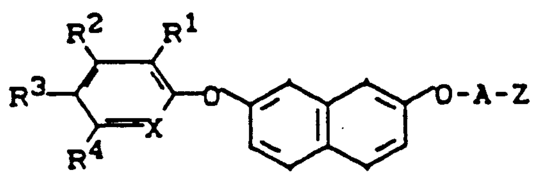

(I)

in which

$R^1$ represents halogen, cyano or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen,

X represents nitrogen or the grouping C-$R^5$, where

$R^5$ represents hydrogen or halogen,

- with the proviso that at least one of the substituents $R^2$ or $R^4$ is different from hydrogen if $R^1$ represents chlorine or cyano and simultaneously $R^3$ represents trifluoromethyl and X represents CH, and that $R^3$ is different from trifluoromethyl if X represents nitrogen -

A represents optionally branched alkanediyl, and

Z represents cyano or the grouping -CO-Y, where

Y represents halogen, hydroxyl, amino, alkylamino, alkenylamino, alkinylamino, arylamino, aralkylamino, alkoxycarbonylalkylamino, cyanoamino, dialkylamino, dialkenylamino, N-alkylarylamino, alkylsulphonylamino, arylsulphonylamino, hydroxyamino, alkoxyamino, hydrazino, alkylsulphonylhydrazino, arylsulphonylhydrazino, alkylthio, arylthio, aralkylthio, alkoxycarbonylalkylthio or the grouping -O-$R^6$, where

$R^6$ represents an optionally halogen-substituted radical from the series comprising alkyl, alkenyl, alkinyl, alkoxyalkyl, alkylthioalkyl, alkylsulphinylalkyl, alkylsulphonylalkyl, aryloxyalkyl, aralkoxyalkyl, trialkylsilylalkyl, arylthioalkyl, aralkylthioalkyl, alkoxycarbonylalkyl, alkylaminocarbonylalkyl, arylaminocarbonylalkyl, N-alkyl-N-aryl-aminocarbonylalkyl, aralkyl, azolylalkyl and alkylideneamino, or an ammonium equivalent, alkylammonium equivalent, alkali metal equivalent or alkaline earth metal equivalent,
or the grouping

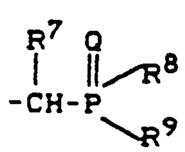

where

$R^7$ represents hydrogen, alkyl, aryl, furyl, thienyl or pyridyl,

$R^8$ represents alkyl or alkoxy,

$R^9$ represents alkoxy and

Q represents oxygen or sulphur,

or

$R^6$ represents the grouping $-(CH_2)_n-R^{10}$, where

$R^{10}$ represents an optionally halogen-substituted and/or alkyl-substituted heterocyclic radical from the series comprising furyl, tetrahydrofuryl, oxotetrahydrofuryl, thienyl,

tetrahydrothienyl, perhydropyranyl, oxazolyl, thiazolyl, thiadiazolyl, dioxolanyl, perhydropyrrolyl, oxoperhydropyrrolyl, pyridinyl and pyrimidinyl, and

n represents the numbers 0, 1 or 2,

characterised in that

(a) 7-(hetero)aryloxy-2-naphthols of the general formula (II)

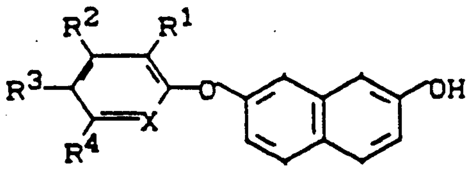

(II)

in which

$R^1$, $R^2$, $R^3$, $R^4$ and X have the abovementioned meanings,

are reacted with carboxylic acid derivatives of the general formula (III)

$Z^1$ - A - Z (III)

in which

A and Z have the abovementioned meanings and

$Z^1$ represents a nucleophilic leaving group,

if appropriate in the presence of an acid acceptor and if appropriate in the presence of a diluent, or

(b) halogeno-(hetero)aryl compounds of the general formula (IV)

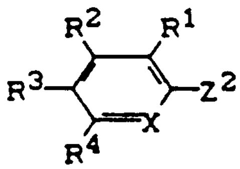

(IV)

in which

$R^1$, $R^2$, $R^3$, $R^4$ and X have the abovementioned meanings and

$Z^2$ represents halogen,

are reacted with (7-hydroxynaphthalen-2-yl-oxy)-alkanecarboxylic acid derivatives of the general formula (V)

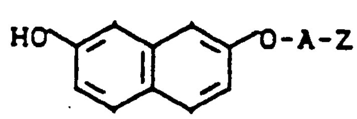

(V)

in which

A and Z have the abovementioned meanings,

if appropriate in the presence of an acid acceptor and if appropriate in the presence of a diluent, or

(c) in the case where in formula (I) Z represents the grouping -CO-Y, where Y represents hydroxyl and A, $R^1$, $R^2$, $R^3$, $R^4$ and X have the abovementioned meanings, compounds of the formula (I) in which Z represents cyano or the grouping -CO-Y, where Y represents methoxy or ethoxy and A, $R^1$, $R^2$, $R^3$, $R^4$ and Y have the abovementioned meanings, are reacted with an alkali metal hydroxide in the presence of an organic solvent, and the reaction mixture is concentrated, if appropriate, and then acidified with a mineral acid, or

(d) in the case where in formula (I) Z represents the grouping -CO-Y, where Y represents halogen and A, $R^1$, $R^2$, $R^3$, $R^4$ and X have the abovementioned meanings, compounds of the general formula (I) in which Z represents the grouping -CO-Y, where Y represents hydroxyl and A, $R^1$, $R^2$, $R^3$, $R^4$ and X have the abovementioned meanings, are reacted with a halogenating agent, if appropriate in the

presence of a diluent, or

(e) in the case where Z in formula (I) represents the grouping -CO-Y, where Y, halogen excepted, has the abovementioned meaning and A, $R^1$, $R^2$, $R^3$, $R^4$ and X have the abovementioned meanings, compounds of the general formula (I), in which Z represents cyano or the grouping -CO-Y, where Y represents halogen and A, $R^1$, $R^2$, $R^3$, $R^4$ and X have the abovementioned meanings, are reacted with compounds of the general formula (VI)

H - Y (VI)

in which

Y ,halogen excepted, has the abovementioned meaning,

if appropriate in the presence of an acid, or an acid acceptor, and if appropriate in the presence of a diluent, or

(f) in the case where Z in formula (I) represents the grouping -CO-Y, where Y represents the grouping $-O-R^6$, where $R^6$ with the exception of ammonium, alkylammonium, alkali metal and alkaline earth metal has the abovementioned meaning and A, $R^1$, $R^2$, $R^3$, $R^4$ and X have the abovementioned meanings, compounds of the general formula (I) in which Z represents the grouping -CO-Y, where Y represents hydroxyl and A, $R^1$, $R^2$, $R^3$, $R^4$ and Y have the abovementioned meanings,

are reacted with compounds of the general formula (VII)

$Z^3 - R^{6-1}$ (VII)

in which

$R^{6-1}$ with the exception of ammonium, alkylammonium, alkali metal and alkaline earth metal has the abovementioned meaning for $R^6$ and

$Z^3$ represents halogen,

if appropriate in the presence of an acid acceptor and if appropriate in the presence of a diluent.

**4.** Herbicidal agents, characterised in that they contain at least one (7-(hetero)aryloxynaphthalen-2-yl-oxy)-alkanecarboxylic acid derivative of the formula (I) according to Claims 1 to 3.

**5.** Method for combating weeds, characterised in that (7-(hetero)aryloxynaphthalen-2-yl-oxy)-alkanecarboxylic acid derivatives of the formula (I) according to Claims 1 to 3 are allowed to act on the weeds and/or their environment.

**6.** Use of (7-(hetero)aryloxynaphthalen-2-yl-oxy)-alkanecarboxylic acid derivatives of the formula (I) according to Claims 1 to 3 for combating weeds.

**7.** Process for the preparation of herbicidal agents, characterised in that (7-(hetero)aryloxynaphthalen-2-yl-oxy)-alkanecarboxylic acid derivatives of the formula (I) according to Claims 1 to 3 are mixed with extenders and/or surfactants.

**8.** The compound of the formula

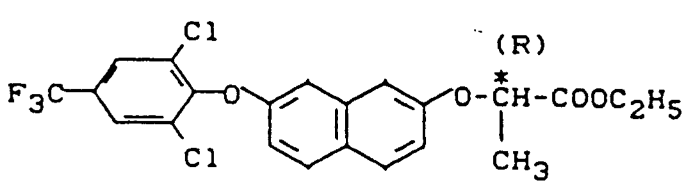

according to Claim 1.

**9.** 7-(Hetero)aryloxy-2-naphthols of the formula (IIa)

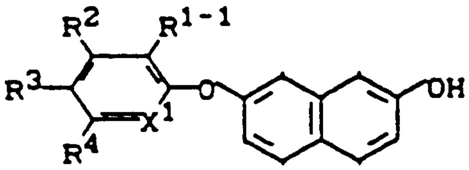

(IIa)

in which

$R^{1-1}$ represents halogen or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen, and

$X^1$ represents nitrogen or the grouping C-$R^{5-1}$, where

$R^{5-1}$ represents halogen.

**10.** Process for the preparation of 7-(hetero)aryloxy-2-naphthols of the formula (IIa)

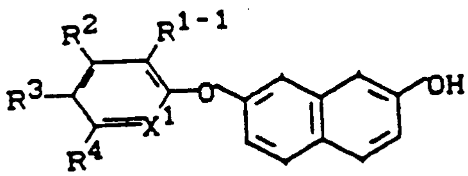

(IIa)

in which

$R^{1-1}$ represents halogen or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen, and

$X^1$ represents nitrogen or the grouping C-$R^{5-1}$, where

$R^{5-1}$ represents halogen,

characterised in that halogeno(hetero)aryl compounds of the formula (IVa),

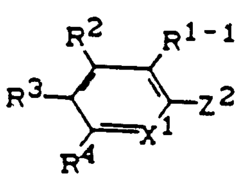

(IVa)

in which

$R^{1-1}$, $R^2$, $R^3$, $R^4$ and $X^1$ have the abovementioned meanings, and

$Z^2$ represents halogen,

are reacted with 2,7-dihydroxynaphthalene in the presence of an acid acceptor and in the presence of a diluent.

## Revendications

**1.** Dérivés d'acides (7-(hétéro)aryloxynaphtalène-2-yl-oxy)-alcanoïques de formule I

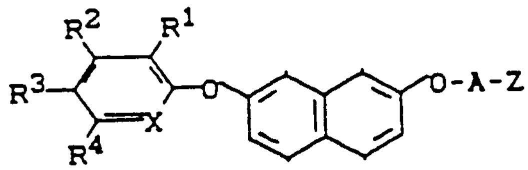

(I)

dans laquelle
$R^1$ représente un halogène, un groupe cyano ou trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène,
X représente l'azote ou le groupement C-$R^5$ dans lequel
$R^5$ représente l'hydrogène ou un halogène,
sous réserve que l'un au moins des symboles $R^2$ et $R^4$ ait une signification autre que l'hydrogène lorsque $R^1$ représente le chlore ou un groupe cyano et que, simultanément, $R^3$ représente un groupe trifluorométhyle et X représente CH, et sous réserve également que $R^3$ ne peut représenter un groupe trifluorométhyle lorsque X représente l'azote,
A représente un groupe alcane-diyle éventuellement ramifié, et
Z représente un groupe cyano ou le groupement -CO-Y dans lequel
Y représente un halogène, un groupe hydroxy, amino, alkylamino, alcénylamino, alcynylamino, arylamino, aralkylamino, alcoxycarbonylalkylamino, cyanamino, dialkylamino, dialcénylamino, N-alkylarylamino, alkylsulfonylamino, arylsulfonylamino, hydroxyamino, alcoxyamino, hydrazino, alkylsulfonylhydrazino, arylsulfonylhydrazino, alkylthio, arylthio, aralkylthio, alcoxycarbonylalkylthio, ou le groupement -O-$R^6$ dans lequel
$R^6$ représente un radical, éventuellement substitué par des halogènes, choisi parmi les radicaux alkyle, alcényle, alcynyle, alcoxyalkyle, alkylthioalkyle, alkylsulfinylalkyle, alkylsulfonylalkyle, aryloxyalkyle, aralcoxyalkyle, trialkylsilylalkyle, arylthioalkyle, aralkylthioalkyle, alcoxycarbonylalkyle, alkylaminocarbonylalkyle, arylaminocarbonylalkyle, N-alkyl-N-arylaminocarbonylalkyle, aralkyle, azolkyle, alkylidène-amino ou un équivalent d'ammonium, d'alkylammonium, de métal alcalin ou alcalinoterreux, ou le groupement

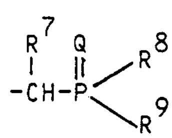

dans lequel
$R^7$ représente l'hydrogène, un groupe alkyle, aryle, furyle, thiényle ou pyridyle,
$R^8$ représente un groupe alkyle ou alcoxy,
$R^9$ représente un groupe alcoxy, et
Q représente l'oxygène ou le soufre,
ou bien
$R^6$ représente le groupement $-(CH_2)_n-R^{10}$ dans lequel
$R^{10}$ représente un radical hétérocyclique éventuellement substitué par des halogènes et/ou des groupes alkyle et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle, et
n est égal à 0, 1 ou 2.

2. Dérivés d'acides (7-(hétéro)aryloxynaphtalène-2-yl-oxy)-alcanoïquesde formule I de la revendication 1, dans laquelle
$R^1$ représente le fluor, le chlore, le brome, un groupe cyano ou trifluorométhyle,
$R^2$ représente l'hydrogène, le fluor ou le chlore,
$R^3$ représente le fluor, le chlore, le brome, un groupe trifluorométhyle, trifluorométhoxy, trifluorométhyl-

thio ou trifluorométhylsulfonyle,

$R^4$ représente l'hydrogène, le fluor ou le chlore,

X représente l'azote ou le groupement C-$R^5$ dans lequel

$R^5$ représente l'hydrogène, le fluor, le chlore ou le brome,

sous réserve qu'au moins des symboles $R^2$ et $R^4$ ait une signification autre que l'hydrogène lorsque $R^1$ représente le chlore ou un groupe cyano et que, simultanément, $R^3$ représente un groupe trifluorométhyle et X représente CH, et sous réserve également que $R^3$ a une signification autre que le groupe trifluorométhyle lorsque X représente l'azote,

A représente un groupe alcane-diyle en $C_1$-$C_4$ éventuellement ramifié, et

Z représente un groupe cyano ou le groupement -CO-Y dans lequel

Y représente le chlore, un groupe hydroxy, amino, alkylamino en $C_1$-$C_6$, alcénylamino en $C_3$-$C_4$, alcynylamino en $C_3$-$C_4$, phénylamino, benzylamino, (alcoxy en $C_1$-$C_4$)-carbonyl-(alkylamino en $C_1$-$C_2$, cyanamino, di-(alkyle en $C_1$-$C_4$)-amino, di-(alcényle en $C_3$-$C_4$)-amino, N-(alkyle en $C_1$-$C_4$)-phénylamino, (alkylsulfonylamino en $C_1$-$C_4$), phénylsulfonylamino, tolylsulfonylamino, hydroxyamino, (alcoxyamino en $C_1$-$C_4$), hydrazino, (alkyle en $C_1$-$C_4$)-sulfonylhydrazino, phénylsulfonylhydrazino, tolylsulfonylhydrazino, alkylthio en $C_1$-$C_4$, phénylthio, benzylthio, (alcoxy en $C_1$-$C_4$)-carbonylalkylthio en $C_1$-$C_2$,

ou bien le groupement -O-$R^6$ dans lequel

$R^6$ représente un radical, éventuellement substitué par le fluor et/ou le chlore et choisi parmi les radicaux alkyle en $C_1$-$C_6$, alcényle en $C_3$-$C_4$, alcynyle en $C_3$-$C_4$, (alcoxy en $C_1$-$C_4$)-alkyle en $C_1$-$C_4$, (alkylthio en $C_1$-$C_4$)-alkyle en $C_1$-$C_4$, (alkyle en $C_1$-$C_4$)-sulfinylalkyle en $C_1$-$C_4$, (alkyle en $C_1$-$C_4$)-sulfonylalkyle en $C_1$-$C_4$, phénoxy-alkyle en $C_1$-$C_3$, tri-(alkyle en $C_1$-$C_2$)-silylalkyle en $C_1$-$C_2$,phénylthioalkyle en $C_1$-$C_3$, benzyloxyalkyle en $C_1$-$C_3$, benzylthioalkyle en $C_1$-$C_3$, (alcoxy en $C_1$-$C_4$)-carbonylalkyle en $C_1$-$C_2$, (alkylamino en $C_1$-$C_4$)-carbonylalkyle en $C_1$-$C_2$, phénylaminocarbonylalkyle en $C_1$-$C_4$, N-(alkyle en $C_1$-$C_4$)-N-phénylaminocarbonylalkyle en $C_1$-$C_4$, benzyle, pyrazolylalkyle en $C_1$-$C_4$, alkylidèneamino en $C_2$-$C_4$ ou un équivalent d'ammonium, d'un alkylammonium en $C_1$-$C_4$, de sodium, de potassium ou de calcium,

ou bien le groupement

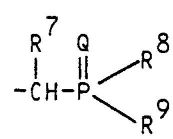

dans lequel

$R^7$ représente l'hydrogène, un groupe alkyle en $C_1$-$C_4$, phényle, furyle, thiényle ou pyridyle,

$R^8$ représente un groupe alkyle en $C_1$-$C_4$ ou alcoxy en $C_1$-$C_4$,

$R^9$ représente un groupe alcoxy en $C_1$-$C_4$, et

Q représente l'oxygène ou le soufre,

ou bien

$R^6$ représente le groupement -$(CH_2)_n$-$R^{10}$ dans lequel

n est égal à 0, 1 ou 2, et

$R^{10}$ représente un radical hétérocyclique, éventuellement subsitué par le fluor, le chlore, le brome et/ou des groupes alkyle en $C_1$-$C_4$, et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle.

**3.** Procédé de préparation des dérivés d'acides (7-(hétéro)aryloxynaphtalène-2-yloxy)-alcanoïques de formule I

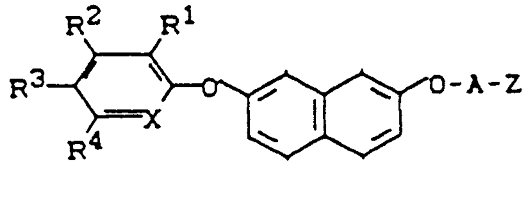

(I)

dans laquelle
$R^1$ représente un halogène, un groupe cyano ou trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène,
X représente l'azote ou le groupement C-$R^5$ dans lequel
$R^5$ représente l'hydrogène ou un halogène,
sous réserve que l'un au moins des symboles $R^2$ et $R^4$ ait une signification autre que l'hydrogène lorsque $R^1$ représente le chlore ou un groupe cyano et que, simultanément, $R^3$ représente un groupe trifluorométhyle et X représente CH, et sous réserve également que $R^3$ ne peut représenter un groupe trifluorométhyle lorsque X représente l'azote,
A représente un groupe alcane-diyle éventuellement ramifié, et
Z représente un groupe cyano ou le groupement -CO-Y dans lequel
Y représente un halogène, un groupe hydroxy, amino, alkylamino, alcénylamino, alcynylamino, arylamino, aralkylamino, alcoxycarbonylalkylamino, cyanamino, dialkylamino, dialcénylamino, N-alkylarylamino, alkylsulfonylamino, arylsulfonylamino, hydroxyamino, alcoxyamino, hydrazino, alkylsulfonylhydrazino, arylsulfonylhydrazino, alkylthio, arylthio, aralkylthio, alcoxycarbonylalkylthio, ou le groupement -O-$R^6$ dans lequel
$R^6$ représente un radical, éventuellement substitué par des halogènes, choisi parmi les radicaux alkyle, alcényle, alcynyle, alcoxyalkyle, alkylthioalkyle, alkylsulfinylalkyle, alkylsulfonylalkyle, aryloxyalkyle, aralcoxyalkyle, trialkylsilylalkyle, arylthioalkyle, aralkylthioalkyle, alcoxycarbonylalkyle, alkylaminocarbonylalkyle, arylaminocarbonylalkyle, N-alkyl-N-arylaminocarbonylalkyle, aralkyle, azolkyle, alkylidène-amino ou un équivalent d'ammonium, d'alkylammonium, de métal alcalin ou alcalinoterreux, ou bien le groupement

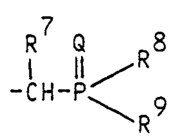

dans lequel
$R^7$ représente l'hydrogène, un groupe alkyle, aryle, furyle, thiényle ou pyridyle,
$R^8$ représente un groupe alkyle ou alcoxy,
$R^9$ représente un groupe alcoxy, et
Q représente l'oxygène ou le soufre,
ou bien
$R^6$ représente le groupement $-(CH_2)_n-R^{10}$ dans lequel
$R^{10}$ représente un radical hétérocyclique éventuellement substitué par des halogènes et/ou des groupes alkyle et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle, et
n est égal à 0, 1 ou 2,
caractérisé en ce que :
a) on fait réagir des 7-(hétéro)aryloxy-2-naphtols de formule générale II

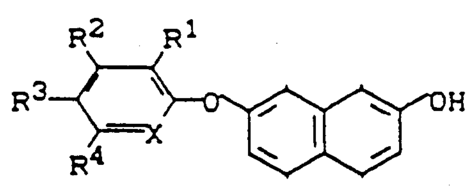

(II)

dans laquelle $R^1$, $R^2$, $R^3$, $R^4$ et X ont les significations indiquées ci-dessus,
avec des dérivés d'acides carboxyliques de formule générale III

$Z^1$ - A - Z (III)

dans laquelle A et Z ont les significations indiquées ci-dessus, et $Z^1$ représente un groupe éliminable nucléophile,
éventuellement en présence d'un accepteur d'acide, et éventuellement en présence d'un diluant, ou bien
b) on fait réagir des dérivés halogéno-(hétéro)aryliques de formule générale IV

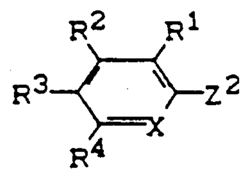
(IV)

dans laquelle $R^1$, $R^2$, $R^3$, $R^4$ et X ont les significations indiquées ci-dessus, et
$Z^2$ représente un halogène,
avec des dérivés d'acides (7-hydroxynaphtalène-2-yloxy)-alcanoïques de formule générale V

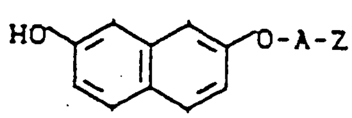
(V)

dans laquelle A et Z ont les significations indiquées ci-dessus,
éventuellement en présence d'un accepteur d'acide et éventuellement en présence d'un diluant, ou bien
c) lorsque, dans la formule I, Z représente le groupement -CO-Y dans lequel Y représente un groupe hydroxy et A, $R^1$, $R^2$, $R^3$, $R^4$ et X ont les significations indiquées ci-dessus, on fait réagir des composés de formule I dans laquelle Z représente un groupe cyano ou le groupement -CO-Y dans lequel Y représente un groupe méthoxy ou éthoxy, et A, $R^1$, $R^2$, $R^3$, $R^4$ et Y ont les significations indiquées ci-dessus, avec un hydroxyde alcalin en présence d'un solvant organique puis, éventuellement après concentration, on acidifie par un acide minéral, ou bien
d) lorsque, dans la formule I, Z représente -CO-Y dans lequel Y représente un halogène, et A, $R^1$, $R^2$, $R^3$, $R^4$ et X ont les significations indiquées ci-dessus, on fait réagir des composés de formule générale I dans laquelle Z représente le groupement -CO-Y dans lequel Y représente un groupe hydroxy, et A, $R^1$, $R^2$, $R^3$, $R^4$ et X ont les significations indiquées ci-dessus, avec un agent halogénant, éventuellement en présence d'un diluant, ou bien
e) lorsque Z dans la formule I, représente le groupement -CO-Y dans lequel Y a les significations indiquées ci-dessus, sauf un halogène, et A, $R^1$, $R^2$, $R^3$, $R^4$ et X ont les significations indiquées ci-dessus,
on fait réagir des composés de formule générale I dans laquelle Z représente un groupe cyano ou le groupement -CO-Y dans lequel Y représente un halogène, et A, $R^1$, $R^2$, $R^3$, $R^4$ et X ont les significations indiquées ci-dessus,
avec des composés de formule générale VI

H - Y (VI)

dans laquelle
Y, à l'exception d'un halogène, a les significations indiquées ci-dessus,
éventuellement en présence d'un acide ou d'un accepteur d'acide et le cas échéant en présence d'un diluant,
ou bien
f) lorsque Z, dans la formule I, représente le groupement -CO-Y dans lequel Y représente le groupement $-O-R^6$ dans lequel $R^6$ a les significations indiquées ci-dessus à l'exception de l'ammonium, d'un alkylammonium, d'un métal alcalin et alcalino-terreux, et A, $R^1$, $R^2$, $R^3$, $R^4$ et X ont les significations indiquées ci-dessus, on fait réagir des composés de formule générale I dans laquelle Z

représente le groupement -CO-Y dans lequel Y représente un groupe hydroxy, et A, $R^1$, $R^2$, $R^3$, $R^4$ et Y ont les significations indiquées ci-dessus,
avec des composés de formule générale VII

$Z^3$ - $R^{6-1}$ (VII)

dans laquelle
$R^{6-1}$ a les significations indiquées ci-dessus pour $R^6$ à l'exception de l'ammonium, d'un alkylammonium, d'un métal alcalin ou alcalinoterreux, et
$Z^3$ représente un halogène,
éventuellement en présence d'un accepteur d'acide et éventuellement en présence d'un diluant.

**4.** Produits herbicides, caractérisés en ce qu'ils contiennent au moins un dérivé d'acide (7-(hétéro)-aryloxynaphtalène-2-yloxy)-alcanoïque de formule I des revendications 1 à 3.

**5.** Procédé pour combattre les végétaux adventices, caractérisé en ce que l'on fait agir sur ces végétaux et/ou leur habitat des dérivés d'acides (7-(hétéro)aryloxynaphtalène-2-yloxy)-alcanoïques de formule I selon revendications 1 à 3.

**6.** Utilisation des dérivés d'acides (7-(hétéro)aryloxynaphtalène-2-yloxy)-alcanoïques de formule I selon revendications 1 à 3 pour la lutte contre les végétaux adventices.

**7.** Procédé de préparation de produits herbicides, caractérisé en ce que l'on mélange des dérivés d'acides (7-(hétéro)aryloxynaphtalène-2-yloxy)-alcanoïques de formule I des revendications 1 à 3, avec des diluants et/ou des agents tensioactifs.

**8.** Le composé de formule

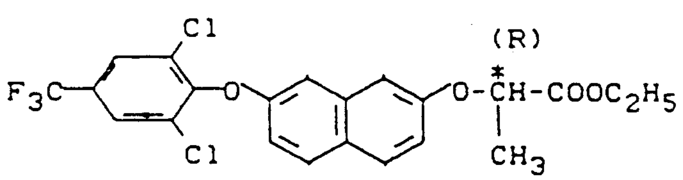

selon revendication 1.

**9.** 7-(hétéro)aryloxy-2-naphtols de formule IIa

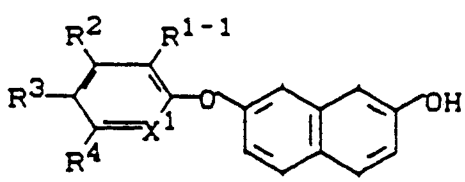

(IIa)

dans laquelle
$R^{1-1}$ représente un halogène ou un groupe trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène,
$X^1$ représente l'azote ou le groupement C-$R^{5-1}$ dans lequel
$R^{5-1}$ représente un halogène.

**10.** Procédé de préparation des 7-(hétéro)aryloxy-2-naphtols de formule IIa

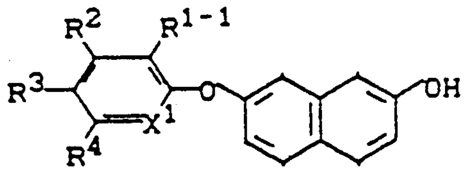

(IIa)

dans laquelle
$R^{1-1}$ représente un halogène ou un groupe trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène,
$X^1$ représente l'azote ou le groupement C-$R^{5-1}$ dans lequel
$R^{5-1}$ représente un halogène,
caractérisé en ce que l'on fait réagir des dérivés halogéno(hétéro)aryliques de formule IVa

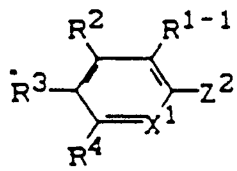

(IVa)

dans laquelle $R^{1-1}$, $R^2$, $R^3$, $R^4$ et $X^1$ ont les significations indiquées ci-dessus, et
$Z^2$ représente un halogène,
avec le 2,7-dihydroxynaphtalène en présence d'un accepteur d'acide et en présence d'un diluant.